(19) Europäisches Patentamt
European Patent Office
Office européen des brevets

(11) **EP 3 538 867 B1**

(12) **EUROPEAN PATENT SPECIFICATION**

(45) Date of publication and mention
of the grant of the patent:
**03.01.2024 Bulletin 2024/01**

(21) Application number: **17869515.1**

(22) Date of filing: **08.11.2017**

(51) International Patent Classification (IPC):
*C12Q 1/6841* (2018.01) *G16B 25/00* (2019.01)

(52) Cooperative Patent Classification (CPC):
(C-Sets available)
**G01N 1/36; C12Q 1/6841** (Cont.)

(86) International application number:
**PCT/US2017/060558**

(87) International publication number:
**WO 2018/089438 (17.05.2018 Gazette 2018/20)**

(54) **MULTIPLEXED IMAGING USING MERFISH, EXPANSION MICROSCOPY, AND RELATED TECHNOLOGIES**

MULTIPLEX-BILDGEBUNG MITTELS MERFISH, EXPANSIONSMIKROSKOPIE UND ZUGEHÖRIGE TECHNOLOGIEN

IMAGERIE MULTIPLEXÉE UTILISANT LA TECHNIQUE MERFISH, LA MICROSCOPIE À EXPANSION ET LES TECHNOLOGIES ASSOCIÉES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.11.2016 US 201662419033 P**
**06.10.2017 US 201762569127 P**

(43) Date of publication of application:
**18.09.2019 Bulletin 2019/38**

(60) Divisional application:
**23207402.1**

(73) Proprietor: **President and Fellows of Harvard College**
**Cambridge, MA 02138 (US)**

(72) Inventors:
• **ZHUANG, Xiaowei**
**Lexington, MA 02421 (US)**
• **MOFFITT, Jeffrey, R.**
**Somerville, MA 02143 (US)**
• **WANG, Guiping**
**Somerville, MA 02143 (US)**

(74) Representative: **Hoffmann Eitle**
**Patent- und Rechtsanwälte PartmbB**
**Arabellastraße 30**
**81925 München (DE)**

(56) References cited:
**US-A1- 2015 087 001     US-A1- 2015 144 490**
**US-A1- 2016 116 384     US-A1- 2016 304 952**
**US-A1- 2016 305 856**

• **FEI CHEN ET AL: "Nanoscale imaging of RNA with expansion microscopy", NATURE METHODS, vol. 13, no. 8, 1 August 2016 (2016-08-01) , pages 679-684, XP055684440, New York ISSN: 1548-7091, DOI: 10.1038/nmeth.3899**
• **NIKOLAY TSANOV ET AL: "smiFISH and FISH-quant - a flexible single RNA detection approach with super-resolution capability", NUCLEIC ACIDS RESEARCH, vol. 44, no. 22, 5 September 2016 (2016-09-05), pages e165-e165, XP055694356, ISSN: 0305-1048, DOI: 10.1093/nar/gkw784**

- F. CHEN ET AL: "Expansion microscopy", SCIENCE, vol. 347, no. 6221, 30 January 2015 (2015-01-30), pages 543-548, XP055511476, US ISSN: 0036-8075, DOI: 10.1126/science.1260088 -& FEI CHEN ET AL: "Supplementary Material for Expansion Microscopy", SCIENCE, vol. 347, no. 6221, 15 January 2015 (2015-01-15), pages 1-18, XP055315649, US ISSN: 0036-8075, DOI: 10.1126/science.1260088
- JEFFREY R. MOFFITT ET AL: "High-performance multiplexed fluorescence in situ hybridization in culture and tissue with matrix imprinting and clearing", PROCEEDINGS OF THE NATIONAL ACADEMY OF SCIENCES, vol. 113, no. 50, 22 November 2016 (2016-11-22), pages 14456-14461, XP055585726, ISSN: 0027-8424, DOI: 10.1073/pnas.1617699113
- CHEN ET AL.: 'Nanoscale imaging of RNA with expansion microscopy' NATURE METHODS vol. 13, 2016, pages 679 - 684, XP055329375
- TILLBERG ET AL.: 'Protein-retention expansion microscopy of cells and tissues labeled using standard fluorescent proteins and antibodies' NAT BIOTECHNOL., [Online] vol. 34, no. 9, September 2016, pages 987 - 992, XP055486308 Retrieved from the Internet: <URL:DOI: 10.1038/nbt.3625> [retrieved on 2016-07-04]
- CHEN ET AL.: 'Expansion microscopy' SCIENCE vol. 347, no. 6221, 30 January 2015, pages 543 - 548, XP055315649

(52) Cooperative Patent Classification (CPC): (Cont.)

C-Sets
**C12Q 1/6841, C12Q 2565/518**

## Description

### RELATED APPLICATIONS

This application claims the benefit of U.S. Provisional Patent Application Serial No. 62/419,033, filed November 8, 2016, entitled "Matrix Imprinting and Clearing," by Zhuang, et al., and U.S. Provisional Patent Application Serial No. 62/569,127, filed October 6, 2017, entitled "Multiplexed Imaging Using MERFISH, Expansion Microscopy, and Related Technologies," by Zhuang, et al.

### GOVERNMENT FUNDING

This invention was made with government support under Grant. No. OD022125 awarded by the National Institutes of Health. The government has certain rights in the invention.

### FIELD

The present invention generally relates to microscopy, and to systems and methods for imaging or determining nucleic acids or other desired targets, for instance, within cells.

### BACKGROUND

Image-based single-cell transcriptomics, in which RNA species are identified, counted and localized in situ via imaging, naturally preserve the native spatial context of RNAs. Multiplexed error-robust fluorescence in situ hybridization (MERFISH) is a method for single-cell transcriptome imaging and has been demonstrated to profile hundreds to thousands of RNAs in single cells. In MERFISH, RNAs are identified via a combinatorial labeling approach that encodes RNA species with error-robust binary barcodes followed by sequential rounds of single-molecule fluorescence in situ hybridization (smFISH) to read out these barcodes. The accuracy of the RNA identification relies on spatially separated signals of individual RNA molecules, which limits the density of RNAs that can be measured and makes the multiplexed imaging of a large number of high abundance RNAs challenging. Accordingly, improvements in imaging techniques are needed. <INSERT PAGE Ia>

US 2016/305856 A1 concerns iterative expansion microscopy.

US 2015/144490 A1 concerns methods and compositions for preparing biological specimens for microscopic analysis.

US 2015/087001 A1 concerns methods for phenotyping of intact whole tissues.

Fei Chen et al., Nature Methods, vol. 13, no. 8, 2016, pages 679-684, concerns nanoscale imaging of RNA with expansion microscopy.

Nikolay Tsanov et al., Nucleic Acids Research, vol. 44, no. 22, 2016, pages e165-e165 concerns smiFISH and FISH-quant - a flexible single RNA detection approach with super-resolution capability.

Fei Chen et al., Science, vol. 347, no. 6221, 2015, pages 543-548, concerns expansion microscopy & Fei Chen et al., Science, vol. 347, no. 6221, 2015, pages 1-18, concerns supplementary material for expansion microscopy.

US 2016/304952 A1 in situ nucleic acid sequencing of expanded biological samples.

### SUMMARY

The present invention is defined by the claims.

The present disclosure generally relates to microscopy, and to systems and methods for imaging or determining nucleic acids or other desired targets, for instance, within cells. The subject matter of the present disclosure involves, in some cases, interrelated products, alternative solutions to a particular problem, and/or a plurality of different uses of one or more systems and/or articles.

In one aspect, the present disclosure is generally directed to an article comprising an expandable material. The material may comprise an embedded cell and a plurality of nucleic acids immobilized to the expandable material. In some cases, at least 50% of the plurality of nucleic acids immobilized to the expandable material are immobilized at single points.

According to another aspect, the present disclosure is generally directed to a polymer comprising an expanded cell and a plurality of nucleic acids immobilized to the polymer. In some embodiments, the cell is expanded to at least 5 times its normal size within the polymer. In certain instances, at least 50% of the plurality of nucleic acids immobilized to the expandable material are immobilized at single points.

The present disclosure, in another aspect, is generally directed to a method. In one set of embodiments, the method comprises embedding cells within an expandable material, immobilizing nucleic acids from the cells to the expandable material, expanding the expandable material, exposing the expandable material to a plurality of nucleic acid probes, and determining binding of the nucleic acid probes to the immobilized nucleic acids.

In another set of embodiments, the method comprises immobilizing a plurality of targets to an expandable material, and expanding the expandable material. In some cases, at least 50% of the plurality of targets immobilized to the expandable material are immobilized at single points.

The method, in yet another set of embodiments, includes immobilizing a plurality of nucleic acids to an expandable material, exposing the expandable material to a plurality of nucleic acid probes, expanding the expandable material, and determining binding of the nucleic acid probes to the immobilized nucleic acids. In certain embodiments, at least 50% of the plurality of nucleic acids immobilized to the expandable material are immobilized at single points.

In another aspect, the present disclosure encompasses methods of making one or more of the embodiments described herein, for example, MERFISH and expansion microscopy. In still another aspect, the present disclosure encompasses methods of using one or more of the embodiments described herein, for example, MERFISH and expansion microscopy.

Other advantages and novel features of the present invention will become apparent from the following detailed description of various non-limiting embodiments of the invention when considered in conjunction with the accompanying figures.

## BRIEF DESCRIPTION OF THE DRAWINGS

Non-limiting embodiments of the present invention will be described by way of example with reference to the accompanying figures, which are schematic and are not intended to be drawn to scale. In the figures, each identical or nearly identical component illustrated is typically represented by a single numeral. For purposes of clarity, not every component is labeled in every figure, nor is every component of each embodiment of the invention shown where illustration is not necessary to allow those of ordinary skill in the art to understand the invention. In the figures:

> Figs. 1A-1E illustrate MERFISH measurements of RNA in an unexpanded sample;
> Figs. 2A-2H illustrate MERFISH measurements of RNA in an expanded sample, in one embodiment of the invention;
> Figs. 3A-3H illustrate imaging in expanded samples in combination with MERFISH, in yet another embodiment of the invention; and
> Figs. 4A-4C schematically illustrate attachment of targets at one or more anchor points.

## DETAILED DESCRIPTION

The present disclosure generally relates to microscopy, and to systems and methods for imaging or determining nucleic acids or other desired targets, for instance, within cells. In certain aspects, a sample is contained within an expandable material, which is expanded and imaged in some fashion. Expansion of the material improves the effective resolution of the subsequent image. This may be combined, for example, with other super-resolution techniques, such as STORM, and/or with techniques such as MERFISH for determining nucleic acids such as mRNA within the sample, for example, by binding nucleic acid probes to the sample. Other aspects are generally directed to compositions or devices for use in such methods, kits for use in such methods, or the like.

In certain aspects, a sample is embedded or contained within an expandable material, such as polyelectrolyte gel. The sample may be, for example, a cell or other biological structure, or a non-biological structure in some cases. Upon expansion of the expandable material, the sample is effectively magnified physically, rather than optically. Components of the sample are separated from each other by expansion of the material, but typically retain comparable geometries or topologies, especially if the expansion of the material occurs substantially isotopically. Thus, components that initially started close to each other are now further apart, and can be more easily detected, e.g., using imaging or other techniques.

Expansion techniques may be combined with techniques for determining nucleic acids such as mRNA within the sample. In some cases, for example, nucleic acids within the sample are attached to the expandable material (for example, covalently) before the expandable material is expanded. The nucleic acids may then be detected, for example, using exposures to nucleic acid probes, e.g., repeatedly, which may be used to determine the distribution of nucleic acids within the material. For example, in techniques such as MERFISH (multiplexed error-robust fluorescence *in situ* hybridization), a sample is exposed to different rounds of nucleic acid probes, and binding of the nucleic acids can be determined using fluorescence or other techniques. In certain cases, a relatively large number of different targets may be identified using a relatively small number of labels, e.g., by using various combinatorial approaches. Identification may also be enhanced in some embodiments using error-checking and/or error-correcting codes. See, e.g., U.S. Patent Application Serial No. 15/329,683, entitled "Systems and Methods for Determining Nucleic Acids," by Zhuang, et al., published as U.S. Patent Application Publication No. 2017/0220733 on August 3, 2017.

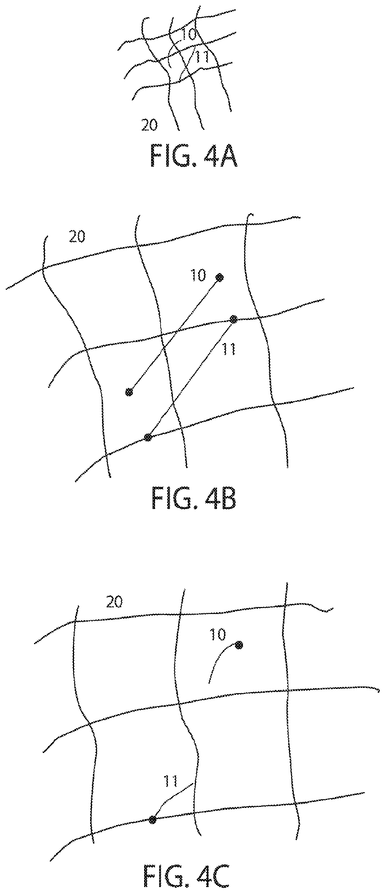

In some cases, one or more targets, for example, nucleic acids such as mRNA, may be immobilized relative to the expandable material prior to expansion. In certain embodiments, targets are attached to the expandable material at a single point. This is important in some embodiments since targets attached at two or more points may be expanded along with the material, which may make it more difficult to resolve targets at a high resolution. For example, referring to Fig. 4A, targets 10 and 11 are shown within an expandable material 20. For example, targets 10 and 11 may be nucleic acids. In Fig. 4B, targets 10 and 11 are attached at two points (e.g., their ends), and upon expansion by the expandable material, targets 10 and 11 are expanded (e.g., "stretched") along with the rest of the expanded material since their anchor points (shown as dots) are expanded along with the expanded material. In contrast, in Fig. 4C, targets 10 and 11 are attached at only one point (shown as a dot), and upon expansion of the expandable material, the targets are not expanded or stretched. Thus, in Fig. 4C, targets 10 and 11 are now further apart, and can be more easily determined, whereas in Fig. 4B targets 10 and 11 may still be physically overlapping and, thus, may produce signals that are not distinguishable despite the expansion process. Accordingly, in certain embodiments of the invention, at least some of the targets, such as nucleic acids, are attached to the expandable material at a single point. In some embodiments, a molecule may behave in the same way as described in Fig. 4C even if it is attached to the gel via multiple attachments, e.g., if the attachments are sufficiently close to one another along the length of the molecule.

In addition, components of the sample that may be contributing to the background, such as proteins, lipids, and/or other non-targets, may be "cleared" from the sample to improve determination in certain cases. However, nucleic acids or other desired targets may be prevented from also being cleared, for example, by attaching the nucleic acids (or other targets) to the expandable material, as noted above. In this way, expandable materials may be combined with techniques such as MERFISH to facilitate detection of mRNA, other nucleic acids, or other targets. Non-limiting examples of such techniques may be found, for example, in U.S. Pat. Apl. Pub. No. 62/419,033, entitled "Matrix Imprinting and Clearing"

A variety of techniques may be used to determine binding, including optical techniques such as fluorescence microscopy. In some cases, spatial positions may be determined at super resolutions, or at resolutions better than the wavelength of light or the diffraction limit (although in other embodiments, super resolutions are not required). For example, techniques such as STORM (stochastic optical reconstruction microscopy) may be used. See, for example, U.S. Pat. No. 7,838,302, issued November 23, 2010, entitled "Sub-Diffraction Limit Image Resolution and Other Imaging Techniques," by Zhuang, et al.

The above discussion is a non-limiting example of one embodiment of the present invention that can be used to determine nucleic acids or other targets in a sample. However, other embodiments are also possible. Accordingly, more generally, various aspects of the disclosure are directed to various systems and methods for imaging or determining nucleic acids or other desired targets, for instance, within cells or other samples.

As mentioned, in one aspect, a sample is embedded or contained within an expandable material. The sample may be any suitable sample, and may be biological in some embodiments. In some cases, the sample contains DNA and/or RNA, e.g., that may be determined within the sample. (In other embodiments, other targets within the sample may be determined.) In some cases, the sample may include cells, such as mammalian cells (including human cells), or other types of cells. The sample may contain viruses in some cases. In addition, in some cases, the sample may be a tissue sample, e.g., from a biopsy, artificially grown or cultured, etc.

In certain embodiments, the expandable material is one that can be expanded, for example, when exposed to water or another suitable liquid. For example, the material may exhibit a relative change in size of at least 1.1, at least 1.2 at least 1.3, at least 1.5, at least 2, at least 3, at least 4, at least 5, at least 7, at least 10, or at least 15, etc., and/or a relative change in size that is less than 15, less than 10, less than 7, less than 5, less than 4, less than 3, less than 2, less than 1.5, less than 1.3, or less than 1.2 (i.e., a change in size of 2 means that a sample doubles in linear dimension), or inverses of these (i.e., an inverse change in size of 2 means that a sample halves in linear dimensions).

In some embodiments, the expandable material may be one that does not significantly distort during the expansion process (e.g., the expandable material may expand substantially uniformly or isotropically in all 3 dimensions), although in some cases, the expandable material may exhibit some distortion or non-isotropic expansion. For example, the expandable material may expand in one dimension, relative to an orthogonal dimension, by less than 150%, less than 130%, less than 125%, less than 120%, less than 115%, less than 110%, or less than 105% by linear dimension relative to the shorter linear expansion.

In some cases, the expandable material is a polymer. Non-limiting examples of suitable polymers include polyelectrolytes and agarose. In some cases, the polymer is a gel or a hydrogel. A variety of polymers can be used in various embodiments including but not limited to acrylic acid, acrylamide, ethylene glycol diacrylate, ethylene glycol dimetharcrylate, poly(ethylene glycol dimethacrylate), poly(N-isopropyl acrylamide), methyl cellulose, (ethylene oxide)-(propylene oxide)-(ethylene oxide) terpolymers, sodium alginate, poly(vinyl alcohol), alginate, chitosan, gum Arabic, gelatin, agarose, or the like. In some cases, the polymer may be selected to be relatively optically transparent. In some cases, the expandable material may be formed from monomers or oligomers, for example, comprising one or more substituted or unsubstituted methacrylates, acrylates, acrylamides, methacrylamides, vinylalcohols, vinylamines, allylamines, allylalcohols, including divinylic crosslinkers thereof (e.g., *N,N*-alkylene bisacrylamides such as *N,N*-methylenebisacrylamide), or the like. In some cases, polymerization initiators and/or crosslinkers may be present. For example, a precursor may include one or more cross-linking agents, which may be used to cross-link a polymeric expandable material as it forms, e.g., during the polymerization process.

In some cases, expansion of the expandable material may be facilitated by exposing the material to water, a solution comprising water, or another suitable medium (e.g., a liquid medium). Without wishing to be bound by any theory, it is believed that water flow into the material may facilitate the expansion of the expandable material. In certain embodiments, the solution may be hypotonic relative to the expandable material, which may facilitate the transport of water into the expandable material, e.g., due to differences in tonicity. Other ways of expanding polymers may be used in other embodiments, such as through changes in the pH, changes in an external electric/magnetic field, changes in temperature, response to light, etc.

In some cases, expansion of the material may be restricted after the expansion has occurred. For example, the material may be stabilized so that exchange of other buffers does not cause a change in size. This stabilization may occur, for example, via chemical modification of the expandable material, or embedding of the expandable material in a second material that is not expandable.

As a non-limiting example, a sample may be exposed to one or more monomers or other precursors which can react to form the expandable material. In some cases, the precursors may be permeated, diffused, or otherwise transported through the sample, before being reacted. For instance, in some cases, a precursor may be present in a liquid (e.g., water, saline, or other aqueous medium), which may permeate through a sample in some fashion.

In some cases, the sample may be embedded within a relatively large polymer or gel, which can then be sectioned or sliced in some cases to produce smaller portions for analysis, e.g., using various microtomy techniques commonly available to those of ordinary skill in the art. For instance, tissues or organs may be immobilized within a suitable polymer or gel.

In certain embodiments, the expansion of the gel may disentangle molecules that were physically overlapping. In a non-limiting example, two RNA molecules may physically overlap within the sample. By anchoring these RNA molecules at one location, the expansion of the gel may separate these molecules so that they are no longer physically overlapping as long as the attachment points for each RNA are sufficiently separated within the sample. In some embodiments, for example, if the RNA molecules are anchored to the gel at multiple locations along their length, then expansion of the gel may not physically separate these two molecules. Rather, expansion of the gel may stretch these molecules between the attachment points, leaving the two regions of these molecules that were overlapping still overlapping after expansion. In this case, these molecules may not be capable of being physically distinguished, e.g., by optical techniques. In some cases, anchoring methods that are deterministic (e.g., that target a specific location of the RNA) versus random (e.g., that target a large number of different locations on the RNA with the specific targeted location selected at random), may differ in the probability that RNAs will be anchored in multiple locations to the gel and, thus, be unable to be physically separated when overlapping. Specific targeting of the 3' or 5' end, or a defined location, within the RNA may in some cases lead to deterministic anchoring that will not stretch RNAs. Moreover, it should be understood that although RNA was used in this example, such considerations also apply to any other biomolecules within samples, e.g. DNA, or other targets described herein.

In one aspect, a sample that is expanded may be imaged or studied to determine nucleic acids or other desired targets, for instance, within cells, tissues or other samples contained within an expandable material. Techniques useful for determining nucleic acids or other desired targets include, but are not limited to, MERFISH, smFISH, or techniques such as those disclosed in U.S. Patent Application Serial No. 15/329,683, filed January 27, 2017, entitled "Systems and Methods for Determining Nucleic Acids," by Zhuang, et al., published as U.S. Patent Application Publication No. 2017/0220733 on August 3, 2017; or U.S. Patent Application Serial No. 15/329,651, filed January 27, 2017, entitled "Probe Library Construction," by Zhuang, et al., published as U.S. Patent Application Publication No. 2017/0212986 on July 27, 2017. In addition, in some cases, a desired target may be immobilized within the expandable material (such as a polymer or gel), while other components are "cleared," e.g., via degradation and/or physical removal, for example, as discussed in U.S. Provisional Patent Application Serial No. 62/419,033, filed November 8, 2016, entitled "Matrix Imprinting and Clearing," by Zhuang, et al.

If nucleic acids are desired to be determined, the nucleic acids may be, for example, DNA, RNA, or other nucleic acids that are present within a cell (or other sample). The nucleic acids may be endogenous to the cell, or added to the cell. For instance, the nucleic acid may be viral, or artificially created. In some cases, the nucleic acid to be determined may be expressed by the cell. The nucleic acid is RNA in some embodiments. The RNA may be coding and/or non-coding RNA. Non-limiting examples of RNA that may be studied within the cell include mRNA, siRNA, rRNA, miRNA, tRNA, lncRNA, snoRNAs, snRNAs, exRNAs, piRNAs, or the like.

In some cases, a significant portion of the nucleic acid within the cell may be studied. For instance, in some cases, enough of the RNA present within a cell may be determined so as to produce a partial or complete transcriptome of the cell. In some cases, at least 4 types of mRNAs are determined within a cell, and in some cases, at least 3, at least 4, at least 7, at least 8, at least 12, at least 14, at least 15, at least 16, at least 22, at least 30, at least 31, at least 32, at least 50, at least 63, at least 64, at least 72, at least 75, at least 100, at least 127, at least 128, at least 140, at least 255, at least 256, at least 500, at least 1,000, at least 1,500, at least 2,000, at least 2,500, at least 3,000, at least 4,000, at least 5,000, at least 7,500, at least 10,000, at least 12,000, at least 15,000, at least 20,000, at least 25,000, at least 30,000, at least 40,000, at least 50,000, at least 75,000, or at least 100,000 types of mRNAs may be determined within a cell.

In some cases, the transcriptome of a cell may be determined. It should be understood that the transcriptome generally encompasses all RNA molecules produced within a cell, not just mRNA. Thus, for instance, the transcriptome may also include rRNA, tRNA, siRNA, etc. In some embodiments, at least 5%, at least 10%, at least 15%, at least 20%, at least 25%, at least 30%, at least 40%, at least 50%, at least 60%, at least 70%, at least 80%, at least 90%, or 100% of the transcriptome of a cell may be determined.

The determination of one or more nucleic acids within the cell or other sample may be qualitative and/or quantitative. In addition, the determination may also be spatial, e.g., the position of the nucleic acid within the cell or other sample may be determined in two or three dimensions. In some embodiments, the positions, number, and/or concentrations of nucleic acids within the cell (or other sample) may be determined.

In some cases, a significant portion of the genome of a cell may be determined. The determined genomic segments may be continuous or interspersed on the genome. For example, in some cases, at least 4 genomic segments are determined within a cell, and in some cases, at least 3, at least 4, at least 7, at least 8, at least 12, at least 14, at least 15, at least 16, at least 22, at least 30, at least 31, at least 32, at least 50, at least 63, at least 64, at least 72, at least 75, at least 100, at least 127, at least 128, at least 140, at least 255, at least 256, at least 500, at least 1,000, at least 1,500, at least 2,000, at least 2,500, at least 3,000, at least 4,000, at least 5,000, at least 7,500, at least 10,000, at least 12,000, at least 15,000, at least 20,000, at least 25,000, at least 30,000, at least 40,000, at least 50,000, at least 75,000, or at least 100,000 genomic segments may be determined within a cell.

In some cases, the entire genome of a cell may be determined. It should be understood that the genome generally encompasses all DNA molecules produced within a cell, not just chromosome DNA. Thus, for instance, the genome may also include, in some cases, mitochondria DNA, chloroplast DNA, plasmid DNA, etc. In some embodiments, at least about 5%, at least about 10%, at least about 15%, at least about 20%, at least about 25%, at least about 30%, at least about 40%, at least about 50%, at least about 60%, at least about 70%, at least about 80%, at least about 90%, or 100% of the genome of a cell may be determined.

However, as discussed, it should be understood that in other embodiments of the invention, other targets may be determined or immobilized, e.g., in addition to and/or instead of nucleic acids. For example, in some embodiments of the invention, the targets to be determined or immobilized may include proteins (e.g., antibodies, enzymes, structural proteins), lipids, carbohydrates, viruses, or the like. In one embodiment, cellular components, such as proteins, can be detected by binding to them proteins, such as antibodies/immunoglobulins (primary antibodies, secondary antibodies, nanobodies, fragments of antibodies, IgG, IgM, IgA, IgD, IgE, etc.), that are conjugated to oligonucleotide probes which are anchored to the polymer or gel. These components could then be removed, leaving the oligonucleotide probes to be detected via hybridization of additional nucleic acid probes, similar or identical to the detection of cellular nucleic acids. In another embodiment, multiple distinct cellular species could be detected simultaneously within the same sample, even if the original components are removed from the gel or polymer. For example, RNA molecules could be detected via hybridization of nucleic acid probes simultaneously with the detection of proteins via antibody-oligonucleotide conjugates, as described above.

As mentioned, the sample can be immobilized or embedded within a polymer or a gel, partially or completely. For example, the sample may be embedded in an expandable material as discussed above. In one set of embodiments, anchor probes may be used during the polymerization process. The anchor probes may include an anchor portion that is able to polymerize with the expandable material, e.g., during and/or after the polymerization process, and a targeting portion that is able to immobilize a target, e.g., chemically and/or physically. For example, in the case of polyacrylamide, the anchor probe may include an acrydite portion that can polymerize and become incorporated into the polymer. As another example, an anchor probe may contain, as a targeting portion, a sequence of nucleic acids that is complementary to a target that is a nucleic acid, such as RNA (e.g., mRNA) or DNA. The targeting portion may be specific to a target, and/or may randomly associate with different targets within a sample (for example, due to non-specific binding). Other portions may be present within the anchor probes as well.

For example, to associate with a target nucleic acid, the anchor probe may comprise a nucleic acid sequence substantially complementary to at least a portion of the target nucleic acid. For instance, the nucleic acid may be complementary to at least 5, 6, 7, 8, 9, 10, 11, 12, 13, 14, 15, 16, 17, 18, 19, or 20 or more nucleotides of the nucleic acid. In some cases the complementarity may be exact (Watson-Crick complementarity), or there may be 1, 2, or more mismatches.

Thus, the anchor probe may contain a portion that can interact with and bind to nucleic acid molecules in some embodiments, and/or other molecules in which immobilization is desired, e.g., proteins or lipids, other desired targets, etc. The immobilization may be covalent or non-covalent. For example, to immobilize a target nucleic acid, the anchor probe may comprise a nucleic acid comprising an acrydite portion (e.g., at the 5' end, the 3' end, an internal base, etc.), and a portion able to recognize the target nucleic acid.

In some cases, the anchor probe can be configured to immobilize mRNA, e.g., in the case of transcriptome analysis. For instance, in one set of embodiments, the anchor probe may contain a plurality of thymine nucleotides, e.g., sequentially, for binding to the poly-A tail of an mRNA. Thus, for example, the anchor probe can have at least 5, 6, 7, 8, 9, 10, 11, 12, 13, 14, 15, 16, 17, 18, 19, or 20 or more consecutive thymine nucleotides (e.g., a poly-dT portion) within the anchor probe. In some cases, at least some of the thymine nucleotides may be "locked" thymine nucleotides. These may comprise at least 20%, at least 30%, at least 40%, at least 50%, at least 60%, at least 70%, or at least 80% of these thymine nucleotides. In certain embodiments, the locked and non-locked nucleotides may alternate. Such locked thymine nucleotides may be useful, for example, to stabilize the hybridization of the poly-A tails of the mRNA with the anchor probe.

In another set of embodiments, the anchor probe may comprise a sequence substantially complementary to mRNA (or another target nucleic acid), as noted above. The sequence may be substantially complementary to all, or only a portion, of the target nucleic acid, for example, an end portion (e.g., towards a 5' end or a 3' end), or a middle portion between the end portions. For example, a nucleic acid may be immobilized using anchor probes having substantially complementary portions to the DNA or RNA target. There may be, e.g., 5 or more, 6 or more, 7 or more, 8 or more, 9 or more, 10 or more, 12 or more, 13 or more, 14 or more, 15 or more, 20 or more, 25 or more, 30 or more, 35 or more, 40 or more, 45 or more, or 50 or more complementary nucleotides between the anchor probe and the nucleic acid.

In another set of embodiments, a plurality of anchor probes may be targeted via the methods described above to each RNA of interests. For example, 2 or more, 3 or more, 4 or more, 5 or more, 10 or more, 15 or more, or 20 or more distinct nucleic acid anchor probes may be targeted to a given RNA. These probes may be targeted to a small region of this RNA such that if multiple probes bind and anchor the RNA to the gel, the majority of the RNA remains unstretched during expansion. If multiple RNA species are targeted at a time, then the number of unique anchor probes applied to the sample may be, for example, 5 or more, 10 or more, 15 or more, 20 or more, 100 or more, 200 or more, 1,000 or more, or 10,000 or more.

Other methods may be used to anchor nucleic acids, or other molecules in which immobilization is desired. In one set of embodiments, nucleic acids such as DNA or RNA may be immobilized by covalent bonding. For example, in one set of embodiments, an alkylating agent may be used that covalently binds to RNA or DNA and contains a second chemical moiety that can be incorporated into the polyelectrolytes as it is polymerized. In yet another set of embodiments, the terminal ribose in an RNA molecule may be oxidized using sodium periodate (or another oxidizing agent) to produce an aldehyde, which may be cross-linked to acrylamide, or other polymer or gel. In other embodiments, chemical agents that are able to modify bases may be used, such as aldehydes, e.g. paraformaldehyde or gluteraldehyde, alkylating agents, or succinimidyl-containing groups; chemical agents that modify the terminal phosphate, such as carbodiimides, e.g., EDC (1-ethyl-3-(3-dimethylaminopropyl)carbodiimide); chemical agents that modify internal sugars, such as p-maleimido-phenyl isocyanate; or chemical agents that modify terminal sugars, such as sodium periodate. In some cases, these chemical agents can carry a second chemical moiety that can then be directly cross-linked to the gel or polymer, and/or which can be further modified with a compound that can be directly cross linked to the gel or polymer.

In still another set of embodiments, the nucleic acids may be physically tangled within the polymer or gel, e.g., due to their length, and, thus, unable to diffuse from their original location within the gel.

Similar anchor probes may be used to immobilize other components to a polymer or gel, in other embodiments. For example, in one set of embodiments, an antibody able to specifically bind to a suitable target (e.g., another protein, a lipid, a carbohydrate, a virus, etc.) may be modified to include an acrydite moiety that can become incorporated within a polymer or gel.

In addition, it should be understood that the embedding of the sample within the expandable material and the immobilization of nucleic acids (or other desired targets) may be performed in any suitable order in various embodiments. For instance, immobilization may occur before, during, or after embedding of the sample. In some cases, the target may be chemically modified or reacted to cross-link to the gel or polymer before or during formation of the gel or polymer.

As mentioned, in some embodiments of the invention, the anchor probes immobilize a target, such as a nucleic acid, to the expandable material at a single point. In some cases, at least 20%, at least 30%, at least 40%, at least 50%, at least 60%, at least 70%, at least 80%, at least 90%, or at least 95% (by number) of the targets immobilized to the expandable material are immobilized at a single point. By using only a single point, relatively large numbers of targets may be immobilized relative to the expandable material, without necessarily affecting function of the targets. For example, multiple points of attachment may potentially disrupt the structure of the target, e.g., due to "stretching" of the target during expansion of the expandable material (see, e.g., Figs. 4A-4C), or due to substitutions or distortions of the target due to the anchor (for example, if the anchoring occurs at or near a site used for binding or structural stability, etc.). However, it should be understood that in some embodiments, targets may be attached to the expandable material at more than one point.

A variety of techniques may be used to attach a target such as a nucleic acid (e.g., RNA or DNA) at a single point. For example, anchor probes may be used that are complementary to specific regions of a nucleic acid (e.g., based on sequence complementarity). In some cases, the anchor probes may be specific to each nucleic acid individually (e.g., having unique nucleic acid sequences that are complementary to that nucleic acid's sequence). Nucleic acids such as DNA or RNA can thus be immobilized to an expandable material, e.g., at a single point or region controlled by the sequence of nucleic acids within the anchor probe. In other embodiments, however, the anchor probes may include nucleic acids that are complementary to a common feature of the nucleic acid, such as the poly-A tail of an mRNA, such that the same anchor probes can immobilize several different nucleic acids (e.g., different mRNA sequences within a sample).

In addition, in some embodiments, more than one type of anchor probe may be used, for example, targeting the same or different portions of a target. For instance, in some embodiments, one or more anchor probes may be applied to a sample such that even if not every target is anchored by every anchor probe, a substantial percentage of targets is immobilized to the expandable material. In some cases, for example, at least 20%, at least 30%, at least 40%, at least 50%, at least 60%, at least 70%, at least 80%, or at least 90% of the targets are immobilized to the expandable material, e.g., via one or more anchor probes.

Other approaches may be used to anchor nucleic acids besides complementary nucleic acid sequences. For example, in one set of embodiments, the 3'OH end of an RNA molecule may be targeted, e.g., chemically, for immobilization to the expandable material. For example, a 3'OH end may be converted into an aldehyde via oxidation with agents such as periodate (e.g., sodium periodate or potassium periodate). The aldehyde can then be reacted with hydrazines, amines, or other moieties that can render the anchor probe capable of being incorporated into the expandable material. For example, a hydrazine or an amine can be linked to chemical moieties such as acrylamide, methacrylamide, or other chemicals capable of being incorporated into an expandable material such as polyacrylamide. Additional examples of attachment techniques have been previously described above. Thus, RNA molecules may in some embodiments be anchored only at their 3'OH ends.

As another non-limiting example, the 3'OH could be a site by which enzymatic extension of the RNA could add an anchor probe. For example, polyA polymerase could be used to add modified A nucleotides to the 3' end of the RNA molecule. These A nucleotides can serve as a binding site for polyT probes as described above. As another example, the A nucleotides may be chemically modified such that they contains an appropriate chemical moiety that can be used to anchor the RNA. For example, adenosine nucleotides that contain the click-chemistry reagent azide can be purchased commercially. This could then be reacted with a DBCO group (dibenzocyclooctyne) that is linked to a methacrylate group via an NHS ester, which can then be incorporated into an expandable material, thereby immobilizing the RNA via the 3'OH end to the expandable material.

As still another example, in some embodiments, the 5' end of an RNA molecule may be targeted, e.g., chemically, for immobilization to the expandable material. For example, carbodiimide groups can be used to react with the 5' phosphate present on uncapped RNA molecules. Capped RNA molecules could be decapped using decapping enzymes such as Dcp2 (mRNA-decapping enzyme 2). For example, cross linkers such as EDC (1-ethyl-3-3-dimethyl-aminopropyl carbodiimide) could be reacted with decapped RNAs to specifically label the 5' end of the RNA. These molecules could then be attached to anchor moieties such as acrylamide or methacrylate, e.g., by reaction with methacrylate molecules containing a primary amine. Such moieties can then be incorporated into an expandable material, thereby immobilizing the RNA via the 5' end to the expandable material.

Other molecules, such as proteins, may also be anchored to the expanded matrix, in various embodiments. For example, proteins could be labeled with reagents that contain anchor moieties. A variety of methods can be used for chemical modification of proteins, including crosslinking to amines via aldehydes, crosslinking to cysteines via disulfide bonds or maleimide, etc. Proteins could also be anchored in some embodiments to the expandable gel via interactions with antibodies, which may be labeled in the above fashion.

After immobilization of nucleic acids, or other suitable molecules, to the polymer or gel, other components within the sample may be "cleared." Such clearance may include removal of the components, and/or degradation of the components (e.g., to smaller components, components that are not fluorescent, etc.) that are not the desired target. In some cases, at least 50%, at least 60%, at least 70%, at least 80%, or at least 90% of the undesired components within the sample may be cleared. Multiple clearance steps can also be performed in certain embodiments, e.g., to remove various undesired components. As discussed, it is believed that the removal of such components may decrease background during analysis (for example, by decreasing background and/or off-target binding), while desired components (such as nucleic acids) can be immobilized and thus not cleared.

For example, proteins may be cleared from the sample using enzymes, denaturants, chelating agents, chemical agents, and the like, which may break down the proteins into smaller components and/or amino acids. These smaller components may be easier to remove physically, and/or may be sufficiently small or inert such that they do not significantly affect the background. Similarly, lipids may be cleared from the sample using surfactants or the like. In some cases, one or more of these are used, e.g., simultaneously or sequentially. Non-limiting examples of suitable enzymes include proteinases such as proteinase K, proteases or peptidases, or digestive enzymes such as trypsin, pepsin, or chymotrypsin. Non-limiting examples of suitable denaturants include guanidine HCl, acetone, acetic acid, urea, or lithium perchlorate. Non-limiting examples of chemical agents able to denature proteins include solvents such as phenol, chloroform, guanidinium isocyananate, urea, formamide, etc. Non-limiting examples of surfactants include Triton X-100 (polyethylene glycol p-(1,1,3,3-tetramethylbutyl)-phenyl ether), SDS (sodium dodecyl sulfate), Igepal CA-630, or poloxamers. Non-limiting examples of chelating agents include ethylenediaminetetraacetic acid (EDTA), citrate, or polyaspartic acid. In some embodiments, compounds such as these may be applied to the sample to clear proteins, lipids, and/or other components. For instance, a buffer solution (e.g., containing Tris or tris(hydroxymethyl)aminomethane) may be applied to the sample, then removed.

Non-limiting examples of DNA enzymes that may be used to remove DNA include DNase I, dsDNase, a variety of restriction enzymes, etc. Non-limiting examples of techniques to clear RNA include RNA enzymes such as RNase A, RNase T, or RNase H, or chemical agents, e.g., via alkaline hydrolysis (for example, by increasing the pH to greater than 10). Non-limiting examples of systems to remove sugars or extracellular matrix include enzymes such as chitinase, heparinases, or other glycosylases. Non-limiting examples of systems to remove lipids include enzymes such as lipidases, chemical agents such as alcohols (e.g., methanol or ethanol), or detergents such as Triton X-100 or sodium dodecyl sulfate. Many of these are readily available commercially. In this way, the background of the sample may be removed, which may facilitate analysis of the nucleic acid probes or other desired targets, e.g., using fluorescence microscopy, or other techniques as discussed herein. As mentioned, in various embodiments, various targets (e.g., nucleic acids, certain proteins, lipids, viruses, or the like) may be immobilized, while other non-targets may be cleared using suitable agents or enzymes. As a non-limiting example, if a protein (such as an antibody) is immobilized, then RNA enzymes, DNA enzymes, systems to remove lipids, sugars, etc. may be used.

In some cases, the desired target is a nucleic acid. In one set of embodiments, as an illustrative non-limiting example, the sample may be studied by exposing it to one or more types of nucleic acid probes, simultaneously and/or sequentially. For instance, in one set of embodiments, the nucleic acid probes may include smFISH or MERFISH probes, such as those discussed in Int. Pat. Apl. Pub. Nos. WO 2016/018960 or WO 2016/018963. However, it should be understood that the following is by way of example only, and in other embodiments, the desired target may be, for example, a protein, a lipid, a virus, or the like.

The nucleic acid probes may comprise nucleic acids (or entities that can hybridize to a nucleic acid, e.g., specifically) such as DNA, RNA, LNA (locked nucleic acids), PNA (peptide nucleic acids), or combinations thereof. In some cases, additional components may also be present within the nucleic acid probes, e.g., as discussed below. Any suitable method may be used to introduce nucleic acid probes into a cell or other sample.

For example, in some embodiments, the cell or other sample is fixed prior to introducing the nucleic acid probes, e.g., to preserve the positions of the nucleic acids within the sample. Techniques for fixing cells and tissues are known to those of ordinary skill in the art. As non-limiting examples, a cell may be fixed using chemicals such as formaldehyde, paraformaldehyde, glutaraldehyde, ethanol, methanol, acetone, acetic acid, or the like. In one embodiment, a cell may be fixed using Hepes-glutamic acid buffer-mediated organic solvent (HOPE). The cells may be fixed, in some embodiments, prior to formation and/or after formation of the expandable material. In some cases, the cells are fixed prior to expansion of the expandable material.

The nucleic acid probes may be introduced into the cell (or other sample) using any suitable method. In some cases, the cell may be sufficiently permeabilized such that the nucleic acid probes may be introduced into the cell by flowing a fluid containing the nucleic acid probes around the cells. In some cases, the cells may be sufficiently permeabilized as part of a fixation process; in other embodiments, cells may be permeabilized by exposure to certain chemicals such as ethanol, methanol, Triton X-100, or the like. In addition, in some embodiments, techniques such as electroporation or microinjection may be used to introduce nucleic acid probes into a cell or other sample.

Certain aspects of the present disclosure are generally directed to nucleic acid probes that are introduced into a cell (or other sample). The probes may comprise any of a variety of entities that can hybridize to a nucleic acid, typically by Watson-Crick base pairing, such as DNA, RNA, LNA, PNA, etc., depending on the application. The nucleic acid probe typically contains a target sequence that is able to bind to at least a portion of a target nucleic acid, in some cases specifically. When introduced into a cell or other sample, the nucleic acid probe may be able to bind to a specific target nucleic acid (e.g., an mRNA, or other nucleic acids as discussed herein). In some cases, the nucleic acid probes may be determined using signaling entities (e.g., as discussed below), and/or by using secondary nucleic acid probes able to bind to the nucleic acid probes (i.e., to primary nucleic acid probes). The determination of such nucleic acid probes is discussed in detail below.

In some cases, more than one type of (primary) nucleic acid probe may be applied to a sample, e.g., simultaneously. For example, there may be at least 2, at least 5, at least 10, at least 25, at least 50, at least 75, at least 100, at least 300, at least 1,000, at least 3,000, at least 10,000, at least 30,000, at least 50,000, at least 100,000, at least 250,000, at least 500,000, or at least 1,000,000 distinguishable nucleic acid probes that are applied to a sample, e.g., simultaneously or sequentially.

The target sequence may be positioned anywhere within the nucleic acid probe (or primary nucleic acid probe or encoding nucleic acid probe). The target sequence may contain a region that is substantially complementary to a portion of a target nucleic acid. In some cases, the portions may be at least 50%, at least 60%, at least 70%, at least 75%, at least 80%, at least 85%, at least 90%, at least 92%, at least 94%, at least 95%, at least 96%, at least 97%, at least 98%, at least 99%, or 100% complementary. In some cases, the target sequence may be at least 5, at least 10, at least 15, at least 20, at least 25, at least 30, at least 35, at least 40, at least 50, at least 60, at least 65, at least 75, at least 100, at least 125, at least 150, at least 175, at least 200, at least 250, at least 300, at least 350, at least 400, or at least 450 nucleotides in length. In some cases, the target sequence may be no more than 500, no more than 450, no more than 400, no more than 350, no more than 300, no more than 250, no more than 200, no more than 175, no more than 150, no more than 125, no more than 100, be no more than 75, no more than 60, no more than 65, no more than 60, no more than 55, no more than 50, no more than 45, no more than 40, no more than 35, no more than 30, no more than 20, or no more than 10 nucleotides in length. Combinations of any of these are also possible, e.g., the target sequence may have a length of between 10 and 30 nucleotides, between 20 and 40 nucleotides, between 5 and 50 nucleotides, between 10 and 200 nucleotides, or between 25 and 35 nucleotides, between 10 and 300 nucleotides, etc. Typically, complementarity is determined on the basis of Watson-Crick nucleotide base pairing.

The target sequence of a (primary) nucleic acid probe may be determined with reference to a target nucleic acid suspected of being present within a cell or other sample. For example, a target nucleic acid to a protein may be determined using the protein's sequence, by determining the nucleic acids that are expressed to form the protein. In some cases, only a portion of the nucleic acids encoding the protein are used, e.g., having the lengths as discussed above. In addition, in some cases, more than one target sequence that can be used to identify a particular target may be used. For instance, multiple probes can be used, sequentially and/or simultaneously, that can bind to or hybridize to different regions of the same target. Hybridization typically refers to an annealing process by which complementary single-stranded nucleic acids associate through Watson-Crick nucleotide base pairing (e.g., hydrogen bonding, guanine-cytosine and adenine-thymine) to form double-stranded nucleic acid.

In some embodiments, a nucleic acid probe, such as a primary nucleic acid probe, may also comprise one or more "read" sequences. However, it should be understood that read sequences are not necessary in all cases. In some embodiments, the nucleic acid probe may comprise 1, 2, 3, 4, 5, 6, 7, 8, 9, 10, 11, 12, 13, 14, 15, 16 or more, 20 or more, 32 or more, 40 or more, 50 or more, 64 or more, 75 or more, 100 or more, 128 or more read sequences. The read sequences may be positioned anywhere within the nucleic acid probe. If more than one read sequence is present, the read sequences may be positioned next to each other, and/or interspersed with other sequences.

The read sequences, if present, may be of any length. If more than one read sequence is used, the read sequences may independently have the same or different lengths. For instance, the read sequence may be at least 5, at least 10, at least 15, at least 20, at least 25, at least 30, at least 35, at least 40, at least 50, at least 60, at least 65, at least 75, at least 100, at least 125, at least 150, at least 175, at least 200, at least 250, at least 300, at least 350, at least 400, or at least 450 nucleotides in length. In some cases, the read sequence may be no more than 500, no more than 450, no more than 400, no more than 350, no more than 300, no more than 250, no more than 200, no more than 175, no more than 150, no more than 125, no more than 100, be no more than 75, no more than 60, no more than 65, no more than 60, no more than 55, no more than 50, no more than 45, no more than 40, no more than 35, no more than 30, no more than 20, or no more than 10 nucleotides in length. Combinations of any of these are also possible, e.g., the read sequence may have a length of between 10 and 30 nucleotides, between 20 and 40 nucleotides, between 5 and 50 nucleotides, between 10 and 200 nucleotides, or between 25 and 35 nucleotides, between 10 and 300 nucleotides, etc.

The read sequence may be arbitrary or random in some embodiments. In certain cases, the read sequences are chosen so as to reduce or minimize homology with other components of the cell or other sample, e.g., such that the read sequences do not themselves bind to or hybridize with other nucleic acids suspected of being within the cell or other sample. In some cases, the homology may be less than 10%, less than 8%, less than 7%, less than 6%, less than 5%, less than 4%, less than 3%, less than 2%, or less than 1%. In some cases, there may be a homology of less than 20 basepairs, less than 18 basepairs, less than 15 basepairs, less than 14 basepairs, less than 13 basepairs, less than 12 basepairs, less than 11 basepairs, or less than 10 basepairs. In some cases, the basepairs are sequential.

In one set of embodiments, a population of nucleic acid probes may contain a certain number of read sequences, which may be less than the number of targets of the nucleic acid probes in some cases. Those of ordinary skill in the art will be aware that if there is one signaling entity and $n$ read sequences, then in general $2^n-1$ different nucleic acid targets may be uniquely identified. However, not all possible combinations need be used. For instance, a population of nucleic acid probes may target 12 different nucleic acid sequences, yet contain no more than 8 read sequences. As another example, a population of nucleic acids may target 140 different nucleic acid species, yet contain no more than 16 read sequences. Different nucleic acid sequence targets may be separately identified by using different combinations of read sequences within each probe. For instance, each probe may contain 1, 2, 3, 4, 5, 6, 7, 8, 9, 10, 11, 12, 13, 14, 15, etc. or more read sequences. In some cases, a population of nucleic acid probes may each contain the same number of read sequences, although in other cases, there may be different numbers of read sequences present on the various probes.

As a non-limiting example, a first nucleic acid probe may contain a first target sequence, a first read sequence, and a second read sequence, while a second, different nucleic acid probe may contain a second target sequence, the same first read sequence, but a third read sequence instead of the second read sequence. Such probes may thereby be distinguished by determining the various read sequences present or associated with a given probe or location, as discussed herein.

In addition, the nucleic acid probes (and their corresponding, complimentary sites on the encoding probes), in certain embodiments, may be made using only 2 or only 3 of the 4 bases, such as leaving out all the "G"s or leaving out all of the "C"s within the probe. Sequences lacking either "G"s or "C"s may form very little secondary structure in certain embodiments, and can contribute to more uniform, faster hybridization.

In some embodiments, the nucleic acid probe may contain a signaling entity. It should be understood that signaling entities are not required in all cases, however; for instance, the nucleic acid probe may be determined using secondary nucleic acid probes in some embodiments, as is discussed in additional detail below. Examples of signaling entities that can be used are also discussed in more detail below.

Other components may also be present within a nucleic acid probe as well. For example, in one set of embodiments, one or more primer sequences may be present, e.g., to allow for enzymatic amplification of probes. Those of ordinary skill in the art will be aware of primer sequences suitable for applications such as amplification (e.g., using PCR or other suitable techniques). Many such primer sequences are available commercially. Other examples of sequences that may be present within a primary nucleic acid probe include, but are not limited to promoter sequences, operons, identification sequences, nonsense sequences, or the like.

Typically, a primer is a single-stranded or partially double-stranded nucleic acid (e.g., DNA) that serves as a starting point for nucleic acid synthesis, allowing polymerase enzymes such as nucleic acid polymerase to extend the primer and replicate the complementary strand. A primer is (e.g., is designed to be) complementary to and to hybridize to a target nucleic acid. In some embodiments, a primer is a synthetic primer. In some embodiments, a primer is a non-naturally-occurring primer. A primer typically has a length of 10 to 50 nucleotides. For example, a primer may have a length of 10 to 40, 10 to 30, 10 to 20, 25 to 50, 15 to 40, 15 to 30, 20 to 50, 20 to 40, or 20 to 30 nucleotides. In some embodiments, a primer has a length of 18 to 24 nucleotides.

In addition, the components of the nucleic acid probe may be arranged in any suitable order. For instance, in one embodiment, the components may be arranged in a nucleic acid probe as: primer-read sequences-targeting sequence-read sequences-reverse primer. The "read sequences" in this structure may each contain any number (including 0) of read sequences, so long as at least one read sequence is present in the probe. Non-limiting example structures include primer-targeting sequence-read sequences-reverse primer, primer-read sequences-targeting sequence-reverse primer, targeting sequence-primer-targeting sequence-read sequences-reverse primer, targeting sequence-primer-read sequences-targeting sequence-reverse primer, primer-target sequence-read sequences-targeting sequence-reverse primer, targeting sequence-primer-read sequence-reverse primer, targeting sequence-read sequence-primer, read sequence-targeting sequence-primer, read sequence-primer-targeting sequence-reverse primer, etc. In addition, the reverse primer is optional in some embodiments, including in all of the above-described examples.

After introduction of the nucleic acid probes into a cell or other sample, the nucleic acid probes may be directly determined by determining signaling entities (if present), and/or the nucleic acid probes may be determined by using one or more secondary nucleic acid probes, in accordance with certain aspects of the disclosure. As mentioned, in some cases, the determination may be spatial, e.g., in two or three dimensions. In addition, in some cases, the determination may be quantitative, e.g., the amount or concentration of a primary nucleic acid probe (and of a target nucleic acid) may be determined. Additionally, the secondary probes may comprise any of a variety of entities able to hybridize a nucleic acid, e.g., DNA, RNA, LNA, and/or PNA, etc., depending on the application. Signaling entities are discussed in more detail below.

A secondary nucleic acid probe may contain a recognition sequence able to bind to or hybridize with a read sequence of a primary nucleic acid probe. In some cases, the binding is specific, or the binding may be such that a recognition sequence preferentially binds to or hybridizes with only one of the read sequences that are present. The secondary nucleic acid probe may also contain one or more signaling entities. If more than one secondary nucleic acid probe is used, the signaling entities may be the same or different.

The recognition sequences may be of any length, and multiple recognition sequences may be of the same or different lengths. If more than one recognition sequence is used, the recognition sequences may independently have the same or different lengths. For instance, the recognition sequence may be at least 5, at least 10, at least 15, at least 20, at least 25, at least 30, at least 35, at least 40, or at least 50 nucleotides in length. In some cases, the recognition sequence may be no more than 75, no more than 60, no more than 65, no more than 60, no more than 55, no more than 50, no more than 45, no more than 40, no more than 35, no more than 30, no more than 20, or no more than 10 nucleotides in length. Combinations of any of these are also possible, e.g., the recognition sequence may have a length of between 10 and 30, between 20 and 40, or between 25 and 35 nucleotides, etc. In one embodiment, the recognition sequence is of the same length as the read sequence. In addition, in some cases, the recognition sequence may be at least 50%, at least 60%, at least 70%, at least 75%, at least 80%, at least 85%, at least 90%, at least 92%, at least 94%, at least 95%, at least 96%, at least 97%, at least 98%, at least 99%, or at least 100% complementary to a read sequence of the primary nucleic acid probe.

As mentioned, in some cases, the secondary nucleic acid probe may comprise one or more signaling entities. Examples of signaling entities are discussed in more detail below.

As discussed, in certain aspects of the disclosure, nucleic acid probes are used that contain various "read sequences." For example, a population of primary nucleic acid probes may contain certain "read sequences" which can bind certain of the secondary nucleic acid probes, and the locations of the primary nucleic acid probes are determined within the sample using secondary nucleic acid probes, e.g., which comprise a signaling entity. As mentioned, in some cases, a population of read sequences may be combined in various combinations to produce different nucleic acid probes, e.g., such that a relatively small number of read sequences may be used to produce a relatively large number of different nucleic acid probes.

Thus, in some cases, a population of primary nucleic acid probes (or other nucleic acid probes) may each contain a certain number of read sequences, some of which are shared between different primary nucleic acid probes such that the total population of primary nucleic acid probes may contain a certain number of read sequences. A population of nucleic acid probes may have any suitable number of read sequences. For example, a population of primary nucleic acid probes may have 1, 2, 3, 4, 5, 6, 7, 8, 9, 10, 11, 12, 13, 14, 15, 16, 17, 18, 19, 20 etc. read sequences. More than 20 are also possible in some embodiments. In addition, in some cases, a population of nucleic acid probes may, in total, have 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 20 or more, 24 or more, 32 or more, 40 or more, 50 or more, 60 or more, 64 or more, 100 or more, 128 or more, etc. of possible read sequences present, although some or all of the probes may each contain more than one read sequence, as discussed herein. In addition, in some embodiments, the population of nucleic acid probes may have no more than 100, no more than 80, no more than 64, no more than 60, no more than 50, no more than 40, no more than 32, no more than 24, no more than 20, no more than 16, no more than 15, no more than 14, no more than 13, no more than 12, no more than 11, no more than 10, no more than 9, no more than 8, no more than 7, no more than 6, no more than 5, no more than 4, no more than 3, or no more than two read sequences present. Combinations of any of these are also possible, e.g., a population of nucleic acid probes may comprise between 10 and 15 read sequences in total.

As a non-limiting example of an approach to combinatorially producing a relatively large number of nucleic acid probes from a relatively small number of read sequences, in a population of 6 different types of nucleic acid probes, each comprising one or more read sequences, the total number of read sequences within the population may be no greater than 4. It should be understood that although 4 read sequences are used in this example for ease of explanation, in other embodiments, larger numbers of nucleic acid probes may be realized, for example, using 5, 8, 10, 16, 32, etc. or more read sequences, or any other suitable number of read sequences described herein, depending on the application. If each of the primary nucleic acid probes contains two different read sequences, then by using 4 such read sequences (A, B, C, and D), up to 6 probes may be separately identified. It should be noted that in this example, the ordering of read sequences on a nucleic acid probe is not essential, i.e., "AB" and "BA" may be treated as being synonymous (although in other embodiments, the ordering of read sequences may be essential and "AB" and "BA" may not necessarily be synonymous). Similarly, if 5 read sequences are used (A, B, C, D, and E) in the population of primary nucleic acid probes, up to 10 probes may be separately identified. For example, one of ordinary skill in the art would understand that, for $k$ read sequences in a population with $n$ read sequences on each probe, up to $\binom{n}{k}$ different probes may be produced, assuming that the ordering of read sequences is not essential; because not all of the probes need to have the same number of read sequences and not all combinations of read sequences need to be used in every embodiment, either more or less than this number of different probes may also be used in certain embodiments. In addition, it should also be understood that the number of read sequences on each probe need not be identical in some embodiments. For instance example, some probes may contain 2 read sequences while other probes may contain 3 read sequences.

In some aspects, the read sequences and/or the pattern of binding of nucleic acid probes within a sample may be used to define an error-detecting and/or an error-correcting code, for example, to reduce or prevent misidentification or errors of the nucleic acids. Thus, for example, if binding is indicated (e.g., as determined using a signaling entity), then the location may be identified with a "1"; conversely, if no binding is indicated, then the location may be identified with a "0" (or vice versa, in some cases). Multiple rounds of binding determinations, e.g., using different nucleic acid probes, can then be used to create a "codeword," e.g., for that spatial location. In some embodiments, the codeword may be subjected to error detection and/or correction. For instance, the codewords may be organized such that, if no match is found for a given set of read sequences or binding pattern of nucleic acid probes, then the match may be identified as an error, and optionally, error correction may be applied sequences to determine the correct target for the nucleic acid probes. In some cases, the codewords may have fewer "letters" or positions that the total number of nucleic acids encoded by the codewords, e.g. where each codeword encodes a different nucleic acid.

Such error-detecting and/or the error-correction code may take a variety of forms. A variety of such codes have previously been developed in other contexts such as the telecommunications industry, such as Golay codes or Hamming codes. In one set of embodiments, the read sequences or binding patterns of the nucleic acid probes are assigned such that not every possible combination is assigned.

For example, if 4 read sequences are possible and a primary nucleic acid probe contains 2 read sequences, then up to 6 primary nucleic acid probes could be identified; but the number of primary nucleic acid probes used may be less than 6. Similarly, for $k$ read sequences in a population with $n$ read sequences on each primary nucleic acid probe, $\binom{n}{k}$ different probes may be produced, but the number of primary nucleic acid probes that are used may be any number more or less than $\binom{n}{k}$. In addition, these may be randomly assigned, or assigned in specific ways to increase the ability to detect and/or correct errors.

As another example, if multiple rounds of nucleic acid probes are used, the number of rounds may be arbitrarily chosen. If in each round, each target can give two possible outcomes, such as being detected or not being detected, up to $2^n$ different targets may be possible for $n$ rounds of probes, but the number of nucleic acid targets that are actually used may be any number less than $2^n$. For example, if in each round, each target can give more than two possible outcomes, such as being detected in different color channels, more than $2^n$ (e.g. $3^n$, $4^n$ ...) different targets may be possible for $n$ rounds of probes. In some cases, the number of nucleic acid targets that are actually used may be any number less than this number. In addition, these may be randomly assigned, or assigned in specific ways to increase the ability to detect and/or correct errors.

For example, in one set of embodiments, the codewords or nucleic acid probes may be assigned within a code space such that the assignments are separated by a Hamming distance, which measures the number of incorrect "reads" in a given pattern that cause the nucleic acid probe to be misinterpreted as a different valid nucleic acid probe. In certain cases, the Hamming distance may be at least 2, at least 3, at least 4, at least 5, at least 6, or the like. In addition, in one set of embodiments, the assignments may be formed as a Hamming code, for instance, a Hamming(7, 4) code, a Hamming(15, 11) code, a Hamming(31, 26) code, a Hamming(63, 57) code, a Hamming(127, 120) code, etc. In another set of embodiments, the assignments may form a SECDED code, e.g., a SECDED(8,4) code, a SECDED(16,4) code, a SCEDED(16, 11) code, a SCEDED(22, 16) code, a SCEDED(39, 32) code, a SCEDED(72, 64) code, etc. In yet another set of embodiments, the assignments may form an extended binary Golay code, a perfect binary Golay code, or a ternary Golay code. In another set of embodiments, the assignments may represent a subset of the possible values taken from any of the codes described above.

For example, a code with the same error correcting properties of the SECDED code may be formed by using only binary words that contain a fixed number of '1' bits, such as 4, to encode the targets. In another set of embodiments, the assignments may represent a subset of the possible values taken from codes described above for the purpose of addressing asymmetric readout errors. For example, in some cases, a code in which the number of '1' bits may be fixed for all used binary words may eliminate the biased measurement of words with different numbers of '1's when the rate at which '0' bits are measured as '1's or '1' bits are measured as '0's are different.

Accordingly, in some embodiments, once the codeword is determined (e.g., as discussed herein), the codeword may be compared to the known nucleic acid codewords. If a match is found, then the nucleic acid target can be identified or determined. If no match is found, then an error in the reading of the codeword may be identified. In some cases, error correction can also be applied to determine the correct codeword, and thus resulting in the correct identity of the nucleic acid target. In some cases, the codewords may be selected such that, assuming that there is only one error present, only one possible correct codeword is available, and thus, only one correct identity of the nucleic acid target is possible. In some cases, this may also be generalized to larger codeword spacings or Hamming distances; for instance, the codewords may be selected such that if two, three, or four errors are present (or more in some cases), only one possible correct codeword is available, and thus, only one correct identity of the nucleic acid targets is possible.

The error-correcting code may be a binary error-correcting code, or it may be based on other numbering systems, e.g., ternary or quaternary error-correcting codes. For instance, in one set of embodiments, more than one type of signaling entity may be used and assigned to different numbers within the error-correcting code. Thus, as a non-limiting example, a first signaling entity (or more than one signaling entity, in some cases) may be assigned as "1" and a second signaling entity (or more than one signaling entity, in some cases) may be assigned as "2" (with "0" indicating no signaling entity present), and the codewords distributed to define a ternary error-correcting code. Similarly, a third signaling entity may additionally be assigned as "3" to make a quaternary error-correcting code, etc.

As discussed above, in certain aspects, signaling entities are determined, e.g., to determine nucleic acid probes and/or to create codewords. In some cases, signaling entities within a sample may be determined, e.g., spatially, using a variety of techniques. In some embodiments, the signaling entities may be fluorescent, and techniques for determining fluorescence within a sample, such as fluorescence microscopy or confocal microscopy, may be used to spatially identify the positions of signaling entities within a cell. In some cases, the positions of entities within the sample may be determined in two or even three dimensions. In addition, in some embodiments, more than one signaling entity may be determined at a time (e.g., signaling entities with different colors or emissions), and/or sequentially.

In addition, in some embodiments, a confidence level for the identified nucleic acid target may be determined. For example, the confidence level may be determined using a ratio of the number of exact matches to the number of matches having one or more one-bit errors. In some cases, only matches having a confidence ratio greater than a certain value may be used. For instance, in certain embodiments, matches may be accepted only if the confidence ratio for the match is greater than about 0.01, greater than about 0.03, greater than about 0.05, greater than about 0.1, greater than about 0.3, greater than about 0.5, greater than about 1, greater than about 3, greater than about 5, greater than about 10, greater than about 30, greater than about 50, greater than about 100, greater than about 300, greater than about 500, greater than about 1000, or any other suitable value. In addition, in some embodiments, matches may be accepted only if the confidence ratio for the identified nucleic acid target is greater than an internal standard or false positive control by about 0.01, about 0.03, about 0.05, about 0.1, about 0.3, about 0.5, about 1, about 3, about 5, about 10, about 30, about 50, about 100, about 300, about 500, about 1000, or any other suitable value

In some embodiments, the spatial positions of the entities (and thus, nucleic acid probes that the entities may be associated with) may be determined at relatively high resolutions. For instance, the positions may be determined at spatial resolutions of better than about 100 micrometers, better than about 30 micrometers, better than about 10 micrometers, better than about 3 micrometers, better than about 1 micrometer, better than about 800 nm, better than about 600 nm, better than about 500 nm, better than about 400 nm, better than about 300 nm, better than about 200 nm, better than about 100 nm, better than about 90 nm, better than about 80 nm, better than about 70 nm, better than about 60 nm, better than about 50 nm, better than about 40 nm, better than about 30 nm, better than about 20 nm, or better than about 10 nm, etc.

There are a variety of techniques able to determine or image the spatial positions of entities or targets optically, e.g., using fluorescence microscopy, using radioactivity, via conjugation with suitable chromophores, or the like. For example, various conventional microscopy techniques that may be used in various embodiments of the invention include, but are not limited to, epi-fluorescence microscopy, total-internal-reflectance microscopy, highly-inclined thin-illumination (HILO) microscopy, light-sheet microscopy, scanning confocal microscopy, scanning line confocal microscopy, spinning disk confocal microscopy, or other comparable conventional microscopy techniques.

In some embodiments, *in situ* hybridization (ISH) techniques for labeling nucleic acids such as DNA or RNA may be used, e.g., where nucleic acid probes may be hybridized to nucleic acids in samples. These may be performed, e.g., at cellular-scale or single-molecule-scale resolutions. In some cases, the ISH probes can be composed of RNA, DNA, PNA, LNA, other synthetic nucleotides, or the like, and/or a combination of any of these. The presence of a hybridized probe can be measured, for example, with radioactivity using radioactively labeled nucleic acid probes, immunohistochemistry using, for example, biotin labeled nucleic acid probes, enzymatic chromophore or fluorophore generation using, for example, probes that can bind enzymes such as horseradish peroxidase and approaches such as tyramide signal amplification, fluorescence imaging using nucleic acid probes directly labeled with fluorophores, or hybridization of secondary nucleic acid probes to these primary probes, with the secondary probes detected via any of the above methods.

In some cases, the spatial positions may be determined at super resolutions, or at resolutions better than the wavelength of light or the diffraction limit (although in other embodiments, super resolutions are not required). Non-limiting examples include STORM (stochastic optical reconstruction microscopy), STED (stimulated emission depletion microscopy), NSOM (Near-field Scanning Optical Microscopy), 4Pi microscopy, SIM (Structured Illumination Microscopy), SMI (Spatially Modulated Illumination) microscopy, RESOLFT (Reversible Saturable Optically Linear Fluorescence Transition Microscopy), GSD (Ground State Depletion Microscopy), SSIM (Saturated Structured-Illumination Microscopy), SPDM (Spectral Precision Distance Microscopy), Photo-Activated Localization Microscopy (PALM), Fluorescence Photoactivation Localization Microscopy (FPALM), LIMON (3D Light Microscopical Nanosizing Microscopy), Super-resolution optical fluctuation imaging (SOFI), or the like. See, e.g., U.S. Pat. No. 7,838,302, issued November 23, 2010, entitled "Sub-Diffraction Limit Image Resolution and Other Imaging Techniques," by Zhuang, et al.; U.S. Pat. No. 8,564,792, issued October 22, 2013, entitled "Sub-diffraction Limit Image Resolution in Three Dimensions," by Zhuang, et al.; or Int. Pat. Apl. Pub. No. WO 2013/090360, published June 20, 2013, entitled "High Resolution Dual-Objective Microscopy," by Zhuang, et al.

In one embodiment, the sample may be illuminated by single Gaussian mode laser lines. In some embodiments, the illumination profiled may be flattened by passing these laser lines through a multimode fiber that is vibrated via piezo-electric or other mechanical means. In some embodiments, the illumination profile may be flattened by passing single-mode, Gaussian beams through a variety of refractive beam shapers, such as the piShaper or a series of stacked Powell lenses. In yet another set of embodiments, the Gaussian beams may be passed through a variety of different diffusing elements, such as ground glass or engineered diffusers, which may be spun in some cases at high speeds to remove residual laser speckle. In yet another embodiment, laser illumination may be passed through a series of lenslet arrays to produce overlapping images of the illumination that approximate a flat illumination field.

In some embodiments, the centroids of the spatial positions of the entities may be determined. For example, a centroid of a signaling entity may be determined within an image or series of images using image analysis algorithms known to those of ordinary skill in the art. In some cases, the algorithms may be selected to determine non-overlapping single emitters and/or partially overlapping single emitters in a sample. Non-limiting examples of suitable techniques include a maximum likelihood algorithm, a least squares algorithm, a Bayesian algorithm, a compressed sensing algorithm, or the like. Combinations of these techniques may also be used in some cases.

In addition, the signaling entity may be inactivated in some cases. For example, in some embodiments, a first secondary nucleic acid probe containing a signaling entity may be applied to a sample that can recognize a first read sequence, then the first secondary nucleic acid probe can be inactivated before a second secondary nucleic acid probe is applied to the sample. If multiple signaling entities are used, the same or different techniques may be used to inactivate the signaling entities, and some or all of the multiple signaling entities may be inactivated, e.g., sequentially or simultaneously.

Inactivation may be caused by removal of the signaling entity (e.g., from the sample, or from the nucleic acid probe, etc.), and/or by chemically altering the signaling entity in some fashion, e.g., by photobleaching the signaling entity, bleaching or chemically altering the structure of the signaling entity, e.g., by reduction, etc.). For instance, in one set of embodiments, a fluorescent signaling entity may be inactivated by chemical or optical techniques such as oxidation, photobleaching, chemically bleaching, stringent washing or enzymatic digestion or reaction by exposure to an enzyme, dissociating the signaling entity from other components (e.g., a probe), chemical reaction of the signaling entity (e.g., to a reactant able to alter the structure of the signaling entity) or the like. For instance, bleaching may occur by exposure to oxygen, reducing agents, or the signaling entity could be chemically cleaved from the nucleic acid probe and washed away via fluid flow.

In some embodiments, various nucleic acid probes (including primary and/or secondary nucleic acid probes) may include one or more signaling entities. If more than one nucleic acid probe is used, the signaling entities may each by the same or different. In certain embodiments, a signaling entity is any entity able to emit light. For instance, in one embodiment, the signaling entity is fluorescent. In other embodiments, the signaling entity may be phosphorescent, radioactive, absorptive, etc. In some cases, the signaling entity is any entity that can be determined within a sample at relatively high resolutions, e.g., at resolutions better than the wavelength of visible light or the diffraction limit. The signaling entity may be, for example, a dye, a small molecule, a peptide or protein, or the like. The signaling entity may be a single molecule in some cases. If multiple secondary nucleic acid probes are used, the nucleic acid probes may comprise the same or different signaling entities.

Non-limiting examples of signaling entities include fluorescent entities (fluorophores) or phosphorescent entities, for example, cyanine dyes (e.g., Cy2, Cy3, Cy3B, Cy5, Cy5.5, Cy7, etc.), Alexa Fluor dyes, Atto dyes, photoswtichable dyes, photoactivatable dyes, fluorescent dyes, metal nanoparticles, semiconductor nanoparticles or "quantum dots", fluorescent proteins such as GFP (Green Fluorescent Protein), or photoactivabale fluorescent proteins, such as PAGFP, PSCFP, PSCFP2, Dendra, Dendra2, EosFP, tdEos, mEos2, mEos3, PAmCherry, PAtagRFP, mMaple, mMaple2, and mMaple3. Other suitable signaling entities are known to those of ordinary skill in the art. See, e.g., U.S. Pat. No. 7,838,302 or U.S. Pat. Apl. Ser. No. 61/979,436. In some cases, spectrally distinct fluorescent dyes may be used.

In one set of embodiments, the signaling entity may be attached to an oligonucleotide sequence via a bond that can be cleaved to release the signaling entity. In one set of embodiments, a fluorophore may be conjugated to an oligonucleotide via a cleavable bond, such as a photocleavable bond. Non-limiting examples of photocleavable bonds include, but are not limited to, 1-(2-nitrophenyl)ethyl, 2□nitrobenzyl, biotin phosphoramidite, acrylic phosphoramidite, diethylaminocoumarin, 1-(4,5-dimethoxy-2-nitrophenyl)ethyl, cyclododecyl (dimethoxy-2-nitrophenyl)ethyl, 4-aminomethyl-3-nitrobenzyl, (4-nitro-3-(1-chlorocarbonyloxyethyl)phenyl)methyl-S-acetylthioic acid ester, (4-nitro-3-(1-thlorocarbonyloxyethyl)phenyl)methyl-3-(2-pyridyldithiopropionic acid) ester, 3-(4,4'-dimethoxytrityl)-1-(2-nitrophenyl)-propane-1,3-diol-[2- cyanoethyl-(N,N-diisopropyl)]-phosphoramidite, 1-[2-nitro-5-(6-trifluoroacetylcaproamidomethyl)phenyl]-ethyl-[2-cyanoethyl-(N,N-diisopropyl)]-phosphoramidite, 1-[2-nitro-5-(6-(4,4'-dimethoxytrityloxy)butyramidomethyl)phenyl]-ethyl-[2-cyanoethyl-(N,N-diisopropyl)]-phosphoramidite, 1-[2-nitro-5-(6-(N-(4,4'-dimethoxytrityl))-biotinamidocaproamidomethyl)phenyl]-ethyl-[2-cyanoethyl-(N,N-diisopropyl)]-phosphoramidite, or similar linkers. In another set of embodiments, the fluorophore may be conjugated to an oligonucleotide via a disulfide bond. The disulfide bond may be cleaved by a variety of reducing agents such as, but not limited to, dithiothreitol, dithioerythritol, beta-mercaptoethanol, sodium borohydride, thioredoxin, glutaredoxin, trypsinogen, hydrazine, diisobutylaluminum hydride, oxalic acid, formic acid, ascorbic acid, phosphorous acid, tin chloride, glutathione, thioglycolate, 2,3-dimercaptopropanol, 2-mercaptoethylamine, 2-aminoethanol, tris(2-carboxyethyl)phosphine, bis(2-mercaptoethyl) sulfone, N,N'-dimethyl-N,N'-bis(mercaptoacetyl)hydrazine, 3-mercaptoproptionate, dimethylformamide, thiopropyl-agarose, tri-n-butylphosphine, cysteine, iron sulfate, sodium sulfite, phosphite, hypophosphite, phosphorothioate, or the like, and/or combinations of any of these. In another embodiment, the fluorophore may be conjugated to an oligonucleotide via one or more phosphorothioate modified nucleotides in which the sulfur modification replaces the bridging and/or nonbridging oxygen. The fluorophore may be cleaved from the oligonucleotide, in certain embodiments, via addition of compounds such as but not limited to iodoethanol, iodine mixed in ethanol, silver nitrate, or mercury chloride. In yet another set of embodiments, the signaling entity may be chemically inactivated through reduction or oxidation. For example, in one embodiment, a chromophore such as Cy5 or Cy7 may be reduced using sodium borohydride to a stable, non-fluorescence state. In still another set of embodiments, a fluorophore may be conjugated to an oligonucleotide via an azo bond, and the azo bond may be cleaved with 2-[(2-N-arylamino)phenylazo]pyridine. In yet another set of embodiments, a fluorophore may be conjugated to an oligonucleotide via a suitable nucleic acid segment that can be cleaved upon suitable exposure to DNAse, e.g., an exodeoxyribonuclease or an endodeoxyribonuclease. Examples include, but are not limited to, deoxyribonuclease I or deoxyribonuclease II. In one set of embodiments, the cleavage may occur via a restriction endonuclease. Non-limiting examples of potentially suitable restriction endonucleases include BamHI, BsrI, NotI, XmaI, PspAI, DpnI, MboI, MnlI, Eco57I, Ksp632I, DraIII, AhaII, SmaI, MluI, HpaI, ApaI, BclI, BstEII, TaqI, EcoRI, SacI, HindII, HaeII, DraII, Tsp509I, Sau3AI, PacI, etc. Over 3000 restriction enzymes have been studied in detail, and more than 600 of these are available commercially. In yet another set of embodiments, a fluorophore may be conjugated to biotin, and the oligonucleotide conjugated to avidin or streptavidin. An interaction between biotin and avidin or streptavidin allows the fluorophore to be conjugated to the oligonucleotide, while sufficient exposure to an excess of addition, free biotin could "outcompete" the linkage and thereby cause cleavage to occur. In addition, in another set of embodiments, the probes may be removed using corresponding "toe-hold-probes," which comprise the same sequence as the probe, as well as an extra number of bases of homology to the encoding probes (e.g., 1-20 extra bases, for example, 5 extra bases). These probes may remove the labeled readout probe through a strand-displacement interaction.

As used herein, the term "light" generally refers to electromagnetic radiation, having any suitable wavelength (or equivalently, frequency). For instance, in some embodiments, the light may include wavelengths in the optical or visual range (for example, having a wavelength of between about 400 nm and about 700 nm, i.e., "visible light"), infrared wavelengths (for example, having a wavelength of between about 300 micrometers and 700 nm), ultraviolet wavelengths (for example, having a wavelength of between about 400 nm and about 10 nm), or the like. In certain cases, as discussed in detail below, more than one entity may be used, i.e., entities that are chemically different or distinct, for example, structurally. However, in other cases, the entities may be chemically identical or at least substantially chemically identical.

Another aspect of the disclosure is directed to a computer-implemented method. For instance, a computer and/or an automated system may be provided that is able to automatically and/or repetitively perform any of the methods described herein. As used herein, "automated" devices refer to devices that are able to operate without human direction, i.e., an automated device can perform a function during a period of time after any human has finished taking any action to promote the function, e.g. by entering instructions into a computer to start the process. Typically, automated equipment can perform repetitive functions after this point in time. The processing steps may also be recorded onto a machine-readable medium in some cases.

For example, in some cases, a computer may be used to control imaging of the sample, e.g., using fluorescence microscopy, STORM or other super-resolution techniques such as those described herein. In some cases, the computer may also control operations such as drift correction, physical registration, hybridization and cluster alignment in image analysis, cluster decoding (e.g., fluorescent cluster decoding), error detection or correction (e.g., as discussed herein), noise reduction, identification of foreground features from background features (such as noise or debris in images), or the like. As an example, the computer may be used to control activation and/or excitation of signaling entities within the sample, and/or the acquisition of images of the signaling entities. In one set of embodiments, a sample may be excited using light having various wavelengths and/or intensities, and the sequence of the wavelengths of light used to excite the sample may be correlated, using a computer, to the images acquired of the sample containing the signaling entities. For instance, the computer may apply light having various wavelengths and/or intensities to a sample to yield different average numbers of signaling entities in each region of interest (e.g., one activated entity per location, two activated entities per location, etc.). In some cases, this information may be used to construct an image and/or determine the locations of the signaling entities, in some cases at high resolutions, as noted above.

The following documents are referred to: International Patent Application No. PCT/US2007/017618, filed August 7, 2007, entitled "Sub-Diffraction Image Resolution and Other Imaging Techniques," by Zhuang, et al., published as WO 2008/091296 on July 31, 2008; U.S. Patent No. 7,776,613, issued August 17, 2010, entitled "Sub-Diffraction Image Resolution and Other Imaging Techniques," by Zhuang, et al.; U.S. Patent No. 7,838,302, issued November 23, 2010, entitled "Sub-Diffraction Image Resolution and Other Imaging Techniques," by Zhuang, et al.; International Patent Application No. PCT/US2015/042559, filed July 29, 2015, entitled "Probe Library Construction," by Zhuang, et al., published as WO 2016/018963 on February 4, 2016; International Patent Application No. PCT/US2015/042556, filed July 29, 2015, entitled "Systems and Methods for Determining Nucleic Acids," by Zhuang, et al., published as WO 2016/018960 on February 4, 2016; U.S. Patent Application Serial No. 15/329,683, filed January 27, 2017, entitled "Systems and Methods for Determining Nucleic Acids," by Zhuang, et al., published as U.S. Patent Application Publication No. 2017/0220733 on August 3, 2017; U.S. Patent Application Serial No. 15/329,651, filed January 27, 2017, entitled "Probe Library Construction," by Zhuang, et al., published as U.S. Patent Application Publication No. 2017/0212986 on July 27, 2017; U.S. Patent Application Serial No. 15/252,307, filed August 31, 2016, entitled "Sub-Diffraction Image Resolution and Other Imaging Techniques," by Zhuang, et al., published as U.S. Patent Application Publication No. 2016/0370295 on December 22, 2016; U.S. Provisional Patent Application Serial No. 62/419,033, filed November 8, 2016, entitled "Matrix Imprinting and Clearing," by Zhuang, et al.; U.S. Provisional Patent Application Serial No. 62/511,920, filed May 26, 2017, entitled "Systems and Methods for High-Throughput Image-Based Screening," by Zhuang, et al.; and U.S. Provisional Patent Application Serial No. 62/569,127, filed October 6, 2017, entitled "Multiplexed Imaging Using MERFISH, Expansion Microscopy, and Related Technologies," by Zhuang, et al.

The following examples are intended to illustrate certain embodiments of the present invention, but do not exemplify the full scope of the invention.

## EXAMPLE 1

Some of the following examples illustrates expansion MERFISH, based on certain embodiments of the invention, which exploit expansion microscopy to substantially increase the total density of RNAs measurable by MERFISH. In these examples, RNAs are anchored to a polymer gel that were expanded 12-fold in volume to physically lower RNA densities. With this, accurate identification of RNAs was demonstrated in a MERFISH library composed of abundant RNAs, the total density of which was >10 fold higher than previously measured RNA libraries.

In addition, some of the following examples illustrates combining immunofluorescence with expansion MERFISH, based on certain embodiments, which exploit acrydite-modified oligo-conjugated antibodies to reveal protein localization and expression together with measurements of RNAs. In these examples, samples were stained with acrydite-modified oligonucleotides-conjugated antibodies prior to proteolysis, providing crosslinking to the expandable gel, and then digested. The linked oligonucleotides were stained to reveal the original location of the antibodies together with MERFISH readout of RNAs.

In situ imaging-based approaches to single-cell transcriptomics allow not only the expression profile of individual cells to be determined, but also the spatial positions of individual RNA molecules to be localized. These approaches provide powerful means to map the spatial organizations of RNAs inside cells and the transcriptionally distinct cells in tissues. Currently available image-based single-cell RNA profiling methods rely on either multiplexed fluorescence *in situ* hybridization (FISH) or in situ sequencing. In particular, multiplexed error-robust FISH (MERFISH), a massively multiplexed form of single-molecule FISH (smFISH), allows RNA imaging at the transcriptomic scale. As a powerful method that images individual RNA molecules inside cells, smFISH provides the precise copy number and spatial organization of RNAs in single cells. MERFISH multiplexes smFISH measurements by labeling RNAs combinatorically with oligonucleotide probes which contain error-robust barcodes and measuring these barcodes through sequential rounds of smFISH imaging. Using this approach, simultaneous imaging of hundreds to thousands of RNA species in individual cells using error detection/correction barcoding schemes have been demonstrated. See, e.g., U.S. Pat. Apl. Pub. No. 2017-0220733, entitled "Systems and Methods for Determining Nucleic Acids," and U.S. Pat. Apl. Pub. No. 2017-0212986, entitled "Probe Library Construction".

The measurement throughput of MERFISH to tens of thousands of cells per single-day-long measurement may also be increased. In addition, sample clearing approaches that increase the signal-to-background ratio by anchoring cellular RNAs to a polymer matrix and removing other cellular components that give rise to fluorescence background have been developed, and this clearing approaches allows high-quality MERFISH measurement of tissue sections. See, e.g., U.S. Pat. Apl. Pub. No. 62/419,033, entitled "Matrix Imprinting and Clearing"; and U.S. Pat. Apl. Pub. No. 62/511,920, entitled "Systems and methods for high-throughput image-based screening".

In order to accurately identify RNA molecules, MERFISH, as well as other multiplexed FISH or in situ sequencing based RNA profiling methods, requires non-overlapping fluorescence signals from individual RNAs. However, due to the diffraction limit, when molecules are sufficiently close to each other, their fluorescent signals will overlap, limiting the density of RNAs that can be imaged simultaneously. This problem can be overcome, for example, by super-resolution imaging methods, either through optical means or through sample expansion. These examples illustrates sample expansion and expansion microscopy (ExM) to substantially improve the ability to resolve nearby molecules.

In this approach, the desired signal is conjugated to an expandable polyacrylamide gel, and then the gel is physically expanded by changing the ionic strength of the buffer, separating molecules that would have otherwise produced overlapping fluorescent signals. These examples demonstrates an approach to combine MERFISH and expansion microscopy. The mRNAs is anchored to an expandable polymer gel though acrydite-modified poly-dT oligonucleotides and imaged a high-abundance RNA library in cultured human osteosarcoma cells (U-2 OS), which contains -130 RNA species. Without gel expansion, these RNAs were not well resolved and hence detected with a relatively low detection efficiency of -15-20%. In contrast, in expanded sample, individual RNA molecules became well resolved, leading to a substantial increase in their detection efficiency. Comparison with smFISH and bulk sequencing results demonstrated that these RNAs in the expanded sample were detected with high accuracy and near 100% efficiency. These examples also demonstrated the ability to do simultaneous MERFISH RNA imaging and immunofluorescence imaging of proteins in these expanded samples.

## EXAMPLE 2

Effect of RNA density on the detection efficiency of MERFISH measurements. To illustrate the effect of RNA density on multiplexed smFISH measurements, a high-abundance 129-RNA library was measured using a previously published 16-bit modified Hamming distance 4 (MHD4) binary codes, which allows error detection and correction. This code includes 140 unique code words, and ~129 of them were used to encode the RNAs. -11 were used as blank controls that do not correspond to any RNA. Among the 129 targeted RNAs, 106 were in the abundance range of 40 - 250 copies per cell, and the remaining 23 spanned an abundance range of 1 - 1000 copies per cell to quantify performance across different abundances. The total abundance of RNAs in this library was 14-fold higher than a 130-RNA library previously measured using the MHD4 code with -80-90% detection efficiency.

In the MERFISH measurements, the RNAs were labeled with two sets of probes. In the first step, each cellular RNA was hybridized with a complex set of oligonucleotide probes termed "encoding probes," which contained targeting sequences that bind cellular RNAs and readout sequences that determines the barcodes of these RNAs. In second step, the readout sequences, and hence the barcodes, were detected through a series of smFISH measurements, each round with one or more readout probe complementary to one or more readout sequence. MERFISH measurements were carried out in U-2 OS cells using matrix-imprinting-based clearing methods. See U.S. Pat. Apl. Pub. No. 62/419,033, entitled "Matrix Imprinting and Clearing". Briefly, the cells were fixed, permeabilized, labeled with encoding probes to the 129 RNA species as well as acrydite-modified poly-dT probes that target polyadenylated (polyA) RNAs. The cells were embedded in a polymer gel and cellular proteins and lipids removed by Proteinase K digestion and detergent extraction, while the polyA RNAs were anchored to the gel through the poly-dT probes. After the clearing, eight rounds of two-color smFISH measurements, each with two readout probes, were carried out to read out the 16-bit barcodes on the RNAs, chemical cleavage was used to remove the fluorophores that were linked to the readout probes between consecutive rounds of smFISH imaging. Because the height of the cells were greater than the thickness of a single optical section, the sample was imaged with multiple z-sections to ensure that >90% RNA molecules within the cells were detected.

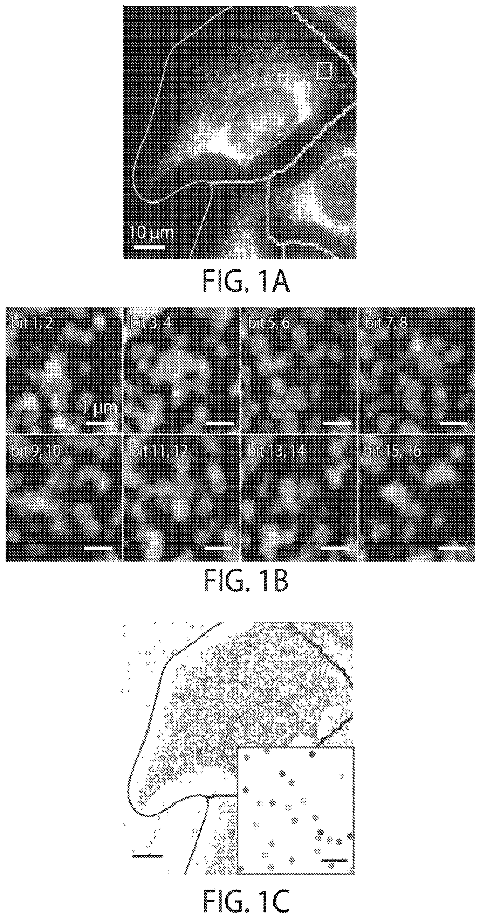

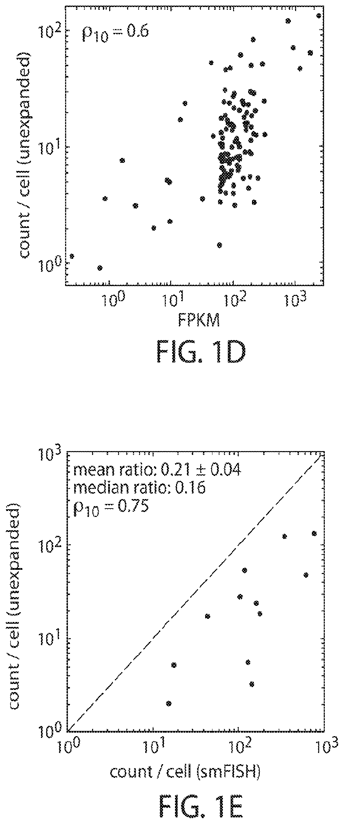

Unlike previous MERFISH measurements on lower abundance RNA libraries because of the high molecular density associated with this 14-fold higher abundance RNA library, a substantial fraction of smFISH signals overlap in space in each round of imaging (Fig. 1A, B). As a result, only a small fraction of the RNA molecules were decodable (Fig. 1C). Comparison with bulk RNA-seq shows that the average copy number per cell detected for these RNAs by MERFISH correlated with the RNA abundance measured by RNA-seq with a Pearson correlation coefficient of 0.6 between the $\log_{10}$ values (Fig. 1D). To determine the detection efficiency, the MERFISH results were compared with the smFISH measurements for 12 genes in this library, also carried out using the matrix-imprinting-based clearing method. This comparison showed that the copy numbers per cell determined by MERFISH were only 21% +/- 4% (average +/- SEM) or 16% (median) of those determined by smFISH (Fig. 1E).

Fig. 1 shows MERFISH measurements of a high-abundance RNA library in unexpanded U-2 OS samples. The samples were cleared using the matrix-imprinting-based clearing method as described above. Fig. 1A shows an image of MERFISH measurement in an unexpanded U-2 OS sample stained with encoding probes for 129 RNAs and visualized with a Cy5-labeled readout probe at one focal plane of the z-scan. The solid lines mark the edge of a cell and the dashed lines mark the DAPI-stained region of a nucleus. Fig. 1B shows high-pass filtered fluorescence images of all 8 rounds of two-color smFISH imaging for the boxed sub-region in (Fig. 1A). Different shadings represent the Cy5 channel (green) and Alexa 750 (red) channel, respectively. Fig. 1C shows the localizations of all decoded RNAs in Fig. 1A based on their measured binary barcodes. The inset shows the localizations of all decoded RNAs of the region shown in Fig. 1B. Decoded RNAs across all optical sections are displayed. The solid lines mark the edge of a cell and the dashed lines mark the DAPI-stained region of a nucleus. Fig. 1D shows the average RNA copy numbers per cell for the 129 RNA species determined by MERFISH vs. the abundances as determined by RNA-seq. The Pearson correlation coefficient between the $\log_{10}$ values ($\rho_{10}$) is 0.6. Fig. 1E shows the average RNA copy numbers per cell determined by MERFISH vs. those by smFISH measurements for 12 of the 129 RNAs. A z-scan of 5 micrometers in depth was performed for each dataset to ensure that at least 90% of the RNA molecules were included in the imaged depth. The Pearson correlation coefficient between the $\log_{10}$ values ($\rho_{10}$) is 0.75. The mean ratio of the copy number values determined by MERFISH to that determined by smFISH is 21% +/- 4% (SEM, $n$ = 12 RNA species) and the median 16%.

## EXAMPLE 3

High RNA-density MERFISH measurements with expansion microscopy. It is believed that the reduced MERFISH detection efficiency may be due, in part, to overlapping single-molecule signals. This problem can be overcome, as is shown in this example, by super-resolution imaging methods, either through optical means or through sample expansion. This example illustrates sample expansion with molecules anchored at a single location and expansion microscopy (ExM) to increase the distance between molecules and substantially improve the ability to resolve nearby molecules.

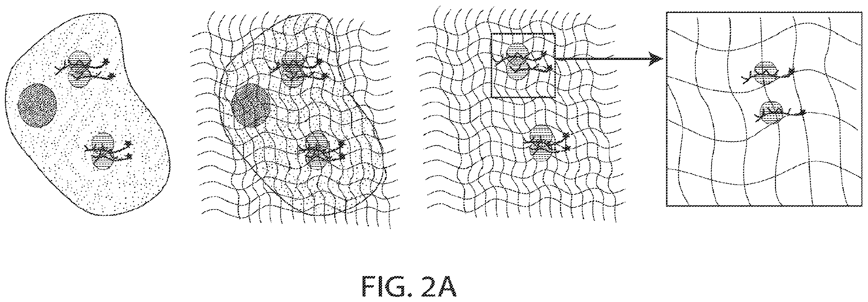

In this example, cells were fixed and permeabilized, and labeled with encoding probes and poly-dT anchoring probes as described above. Then the cells were embedded in an expandable polymer gel. Afterwards, cells were digested by Proteinase K to remove proteins and homogenize the mechanical properties of the gel. The gel was then expanded in low salt buffer and finally embedded again in a non-expandable gel to stabilize it in the expanded state (Fig. 2A). The RNA was anchored to the gel at its poly(A) tail, which creates a single-pointed contact between individual RNA molecules and the gel and avoids stretching the mRNA during expansion process. This should facilitate separation of the nearby molecules. MERFISH encoding probes were stained before embedding samples in a gel since it was found that MERFISH probes did not penetrate reliably well after samples were embedded twice, at least in some embodiments. A low salt buffer (0.5x saline-sodium citrate (SSC)) was used instead of water for dialysis to preserve specific binding of probes during expansion, which resulted in a lower expansion factor. Measurements of the gel volume showed that dialysis in 0.5x saline-sodium citrate (SSC) produced a 12-fold expansion in volume. It was noted that as long as the expansion is sufficient to separate neighboring RNA molecules, a lower expansion factor had the advantage of allowing faster imaging, in certain applications.

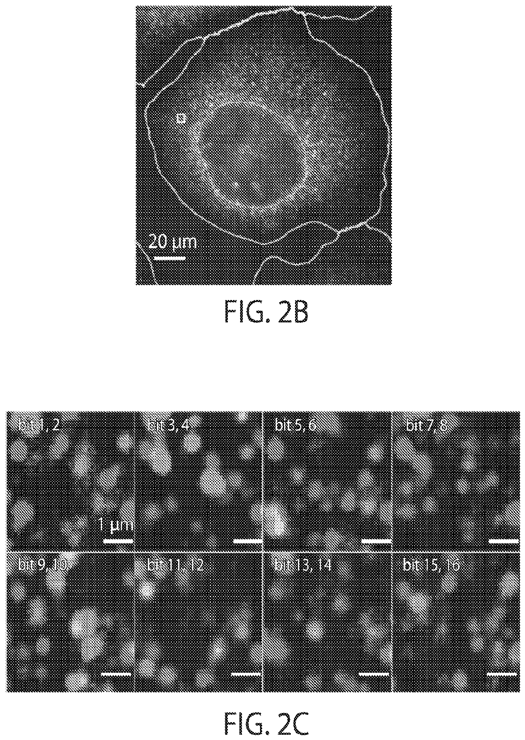

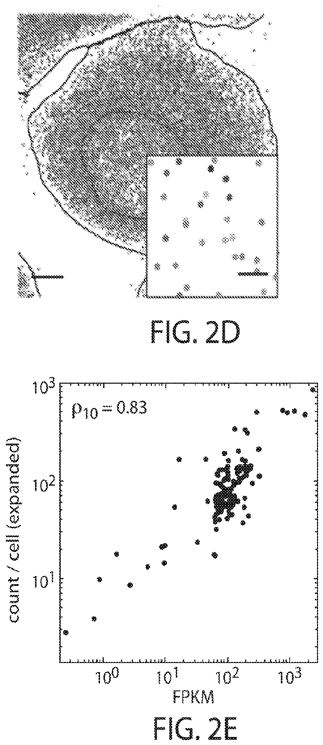

Notably, in the expanded samples in these experiments, individual RNA molecules became well resolved (Fig. 2B, C) and were successfully decoded (Fig. 2D). The average copy number per cell detected for these RNAs by MERFISH correlated with the RNA abundance measured by RNA-seq with a Pearson correlation coefficient of 0.83 between the $\log_{10}$ values (Fig. 2E).

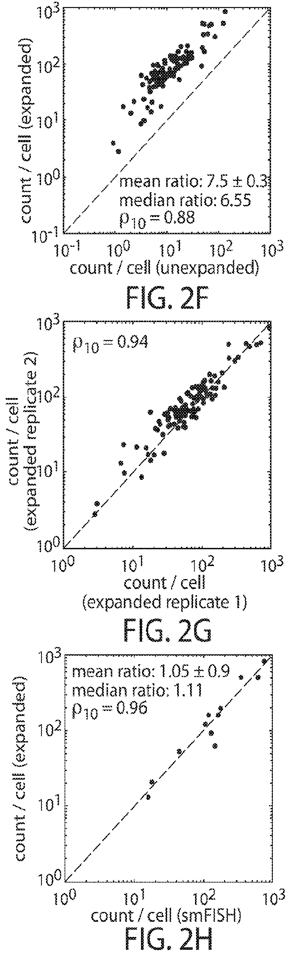

To quantify the improvement on decoding performance with expansion, the average MERFISH counts per RNA species per cell were compared between expanded and unexpanded samples. It was found that the copy numbers per cell for the 129 RNA species detected in expanded samples correlated strongly with those in the unexpanded samples with a high Pearson correlation coefficient of 0.88 between the $\log_{10}$ values (Fig. 2F). However, the copy numbers per cell detected in expanded samples were 7.5 +/- 0.3 fold (average +/-SEM) or 6.6 fold (median) higher than those detected in unexpanded samples for these 129 RNA species, indicating that the detection efficiency for the expanded sample was substantially higher compared to the unexpanded sample for this high-abundance library. The copy number per cell results were highly reproducible between replicates of experiments (Fig.2G). Comparison with smFISH measurements showed that the copy numbers per cell determined by MERFISH was 105% +/- 9% (average +/-SEM) or 111% (median) of those determined by smFISH (Fig. 2H), indicating that the detection efficiency after expansion was close to 100%.

Fig. 2 shows MERFISH measurements of a high-abundance RNA library in expanded U-2 OS cells. Fig.2 A shows a schematic representation of the basic implementation of expansion MERFISH. Target RNAs were co-stained with acrydite-modified poly-dT anchoring probes and MERFISH encoding probes, and then anchored to an expandable polymer gel via the poly-dT probes. Afterwards, samples were incubated in digestion buffer with Proteinase K and SDS for removal of proteins and lipids respectively to homogenize mechanical properties of the gel. Finally, the gel was then expanded by dialysis in low salt buffer and the expanded gel was embedded polyacrylamide gel again to stabilize it in the expanded state. Fig. 2B shows an image of MERFISH measurement in a U-2 OS sample stained with encoding probes for 129 RNAs, embedded, cleared, expanded, re-embedded and visualized with a Cy5-labeled readout probe at one focal plane of the z-scan. The solid lines mark the edge of a cell and the dashed lines mark the DAPI-stained region of a nucleus. Fig. 2C shows a high-pass filtered fluorescence images of all 8 rounds of two-color smFISH imaging for the boxed sub-region in Fig. 2B. Shading represent the Cy5 channel (green) and Alexa 750 channel (red). Fig. 2D shows the localizations of all decoded RNAs in Fig. 2B colored according to their measured binary barcodes. The inset shows the localizations of all decoded RNAs from the region shown in Fig. 2C. Decoded RNAs across all optical sections are displayed. The solid lines mark the edge of a cell and the dashed lines mark the DAPI-stained region of a nucleus.

Fig. 2E shows the average RNA copy numbers per cell for the 129 RNA species determined by MERFISH vs. the abundances as determined by RNA-seq. The Pearson correlation coefficient between the $\log_{10}$ values ($\rho_{10}$) is 0.83. Fig.2 F shows the average RNA copy numbers per cell for the 129 RNA species determined by expansion MERFISH vs. those by MERFISH in unexpanded samples. A z-scan of 12 micrometers in depth was performed for expansion MERFISH measurements and a z-scan of 5 micrometers in depth was performed for unexpanded samples to ensure that at least 90% of the RNA molecules were included in the imaged depth. The Pearson correlation coefficient between the $\log_{10}$ values ($\rho_{10}$) is 0.88. The copy numbers per cell detected in expanded samples were 7.5 +/- 0.3 fold (average +/- SEM) or 6.6 fold (median) of those detected in unexpanded samples for these 129 RNA species. Fig. 2G shows the average RNA copy numbers per cell for the 129 RNA species detected in one expanded sample vs. a second one. The Pearson correlation coefficient between the log10 values ($\rho_{10}$) is 0.94. Fig. 2H shows the average RNA copy numbers per cell determined by expansion MERFISH vs. those by smFISH measurements of cleared and unexpanded samples for 12 of the 129 RNAs. smFISH measurements were performed for the unexpanded but clear sample as shown in Fig. 1. The Pearson correlation coefficient between the $\log_{10}$ values ($\rho_{10}$) is 0.96. The mean ratio between MERFISH and smFISH results was 105% +/- 9% (SEM, *n* = 12 RNA species) and the median ratio 111%.

**EXAMPLE 4**

Incorporating immunofluorescence imaging into MERFISH experiments of expanded samples. A cell is a highly complex system composed of different structures and compartments and immunofluorescence is a powerful technique to visualize specific subcellular structures and compartments. This example thus tested whether combination of immunofluorescence and MERFISH imaging was possible in the expanded samples. Expansion microscopy has been demonstrated in immunostained samples, using oligo-conjugated antibodies and complementary probes with a methacryloyl group to incorporate signals into the gel. These oligos then mark the positions of the protein target and can be detected by hybridization of fluorophore-labeled complementary oligos, which were incorporated into the MERFISH readout measurements. In the experimental procedure, immunostaining was performed of the protein targets with oligo-labeled (secondary) antibodies after hybridization of the cells with the MERFISH encoding probes and RNA anchor probes, and then the labeled sample was embedded in an expandable gel. Acrydite modification was added to the oligo on the antibodies so that it could be incorporated into the polymer gel during the embedding step. After digestion, gel expansion and second embedding in a non-expandable gel as described above, the MERFISH readout procedure was performed to first detect the readout sequences in the RNA encoding probes and then with an additional round of FISH detection to read out the oligo sequences representing the protein target.

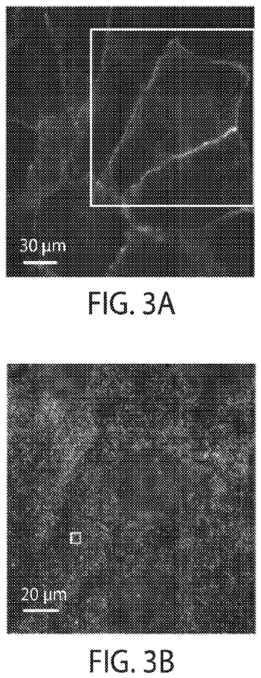

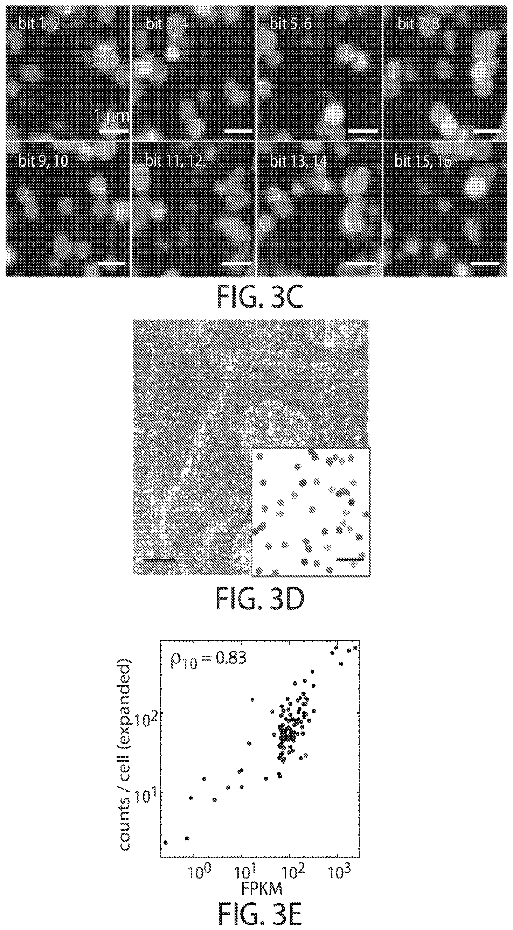

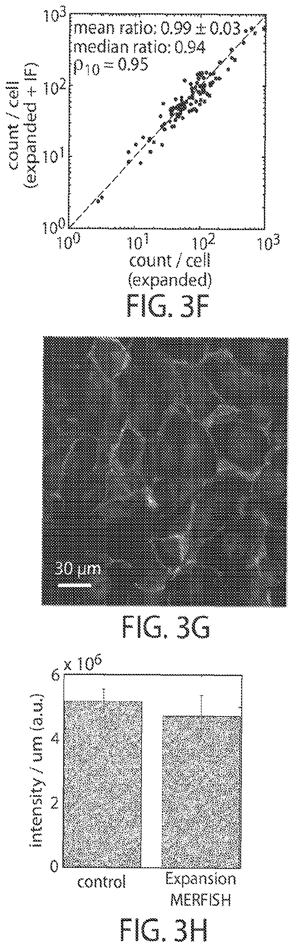

For this example, cadherin was immunostained in cultured U-2 OS cells, which were stained with the same high-abundance MERFISH library described above. Both specific staining of cadherin on the cell periphery (Fig. 3A) as well as clearly resolved smFISH spots in the same cells (Fig. 3B-D) were observed. The combination with immunofluorescence did not affect MERFISH imaging quality (Figs. 3E and 3F). The same high correlation of MERFISH results and the RNA-seq results with a Pearson correlation coefficient of 0.83 between the $\log_{10}$ values (Fig. 3E) was observed. The average copy numbers per RNA species per cell detected in immunostained samples correlated strongly with those in detected samples not subjected to immunostaining, with a Pearson correlation coefficient of 0.95 between the $\log_{10}$ values (Fig. 3F). On average, the ratio of copy numbers per RNA species per cell between immunostained and non-immunostained samples is 99% +/- 3% (SEM), indicating negligible impairment in performance of MERFISH when combined with immunofluorescence. The staining of cadherin in expanded and MERFISH labeled samples also looked similar to cadherin staining in control U-2 OS samples immuostained directly after fixation without MERFISH imaging of the RNA (Fig. 3G). To quantify whether antibody staining was affected by MERFISH RNA labeling, gel embedding, digestion, and expansion procedures, the cadherin intensity per unit length on the cell periphery (normalized to the actual length before expansion) was compared in expansion MERFISH samples versus control samples, with comparable results (Fig. 3H).

Fig. 3 shows incorporating immunofluorescence imaging into MERFISH measurements of expanded samples. Fig. 3A shows an image of an expanded U-2 OS sample stained with MERFISH encoding probes, cadherin primary antibodies, oligo-conjugated secondary antibodies and a Cy5-conjugated complementary probe. Fig. 3B shows smFISH image of MERFISH measurement for the boxed sub-region in Fig. 3A, visualized with a Cy5-labeled readout probe at one focal plane of the z-scan. Fig. 3C shows a high-pass filtered fluorescence images of all 8 rounds of two-color smFISH imaging for the boxed sub-region in Fig. 3B. Shading represent the Cy5 channel (green) and Alexa 750 channel (red). Fig. 3D shows the localizations of all decoded RNAs in Fig. 3B colored according to their measured binary barcodes. The inset shows the localizations of all decoded RNAs of the region shown in Fig. 3C. Decoded RNAs across all optical sections are displayed. Fig. 3E shows the average RNA copy numbers per cell for the 129 RNA species determined by MERFISH vs. the abundances as determined by RNA-seq. The Pearson correlation coefficient between the $\log_{10}$ values ($\rho_{10}$) was 0.83. Fig. 3F shows the average RNA copy numbers per cell for the 129 RNA species determined by expansion MERFISH in immunostained samples vs. those in MERFISH-only samples. A z-scan of 12 micrometer in depth was performed for both. The Pearson correlation coefficient between the $\log_{10}$ values ($\rho_{10}$) was 0.95. The mean ratio between the RNA copy numbers determined in the two experiments was 99% +/- 3% (SEM, $n$ = 129 RNA species) and the median ratio 94%. Fig. 3G shows an image of unexpanded cultured U-2 OS cells stained with cadherin primary antibodies, oligo-conjugated secondary antibodies and a Cy5-conjugated complementary probe right after fixation and permeabilization. Fig. 3H shows cadherin intensity per unit length (normalized to the actual length before expansion) in expanded MERFISH and control samples. Intensities were combined across stacks of z-scan after removal of background.

## EXAMPLE 5

These examples have demonstrated an approach to combine MERFISH and expansion microscopy to measure high-abundance RNA libraries. It was shown that when the total RNA density is high, the overlap between signals from nearby RNA molecules reduced the MERFISH detection efficiency and that sample expansion could overcome this overlapping problem and substantially increase the detection efficiency, recovering the near 100% detection efficiency of MERFISH for these high-abundance RNA libraries.

Each mammalian cell has tens of thousands of different RNA species. MERFISH imaging can be used to determine thousands of RNA species in single cells. To increase the number of RNA species that can be simultaneously imaged in single cells while maintaining similar number of imaging rounds, the density of RNAs imaged per round may increase and may likely become an important limiting factor in the number of RNAs that can be imaged. Expansion MERFISH approach can facilitate a substantial increase in the number of RNA species that can be measured in single cells.

In these examples, the combination of MERFISH with immunofluorescence in these expanded samples was demonstrated, which can provide important information on the cellular context for transcriptome analysis. In conventional immunofluorescence with fluorophore-conjugated antibodies, the number of spectrally-distinct fluorophores limits the number of targets that can be studied simultaneously. The use of oligo-conjugated antibodies potentially allows visualizing many proteins in sequential rounds of hybridization and imaging using just one or a few spectrally distinct fluorophores. Thus, the combination of MERFISH and immunofluorescence imaging may allow a combined proteomic and transcriptomic measurements simultaneously in single cells.

## EXAMPLE 6

This example describes various materials and methods used in some of the above examples.

Design of the encoding probes. The MERFISH-encoding probes were designed using 16-bit Hamming-weight-4 Hamming-distance-4 code with 140 possible barcodes. In this encoding scheme, all barcodes used were separated by a Hamming distance of at least 4, and hence at least four bits must be read incorrectly to change one valid barcode to another. Constant Hamming weight (i.e. the number of "1" bits in each barcodes) of 4 is used to avoid potential measurement bias due to differential rate of "1" to "0" and "0" to "1" errors. The encoding probe set contained 92 encoding probes per RNA. Each encoding probe was comprised of a 30-nt target region designed using a pipeline, flanked by two 20-nt readout sequences randomly selected out of the four ones assigned to each RNA, one 20-nt priming region at the 5' end and another 20-nt priming region using the reverse complement of the T7 promoter at the 3' end. Additional adenosine nucleotide spacers were added between readout sequences and target regions to prevent terminal guanine triplets in the readout sequences and to prevent target regions from combining with Gs from adjacent sequences to form G quadruplets. The priming region at the 5' end had a thymine at the end, which was put at the junction of the priming regions at the 5' end and the encoding region. This was designed to incorporate a uracil on the forward primer used in the reverse transcription step of probe construction, which can be cleaved by Uracil-Specific Excision Reagent (USER) Enzyme. The cleavable primer design together with using reverse complement of the T7 promoter as a second primer allowed construction of encoding probes with a length of 72 nt. The reduction in probe length may facilitate penetration of probes and reduce non-specific binding.

To include some abundant but short RNAs in our library, instead of designing probes targeting distinct regions of RNAs, the probes were allowed to share up to 20 nt with another probe. This modification helped increase the number of probes that could be designed for each gene by 2 fold. 92 target regions per RNA were selected randomly out of all potential target regions of an RNA. By allowing up to a 20-nt overlap between neighboring encoding probes, this design allowed RNAs as short as 1,200 nt to be targeted by 92 encoding probes with a 30-nt targeting sequence. Because a given cellular RNA is typically bound by less than one third of the 92 encoding probes, the encoding probes with overlapping targeting regions were expected to not substantially interfere with each other, but would partially compensate for reduced binding due to local inaccessible regions on the target RNA (e.g. secondary structure) or loss of probe during synthesis.

Construction of the encoding probes. The encoding probe set was constructed from complex oligonucleotide pools. Briefly, the oligopools (CustomArray) were amplified via limited-cycle PCR to make in vitro transcription templates, converted these templates into RNA via in vitro transcription (New England Biolabs), and converted the RNA back to DNA via reverse transcription (Maxima RT H, Thermo Fisher Scientific). The probes were then digested by USER Enzyme (New England Biolabs) at a dilution of 1:30 (vol/vol) incubated at 37 °C for 24 h to cleave off the priming region at the site of a uracil between a priming regions at the 5' end and the target region. After that, DNA was purified via alkaline hydrolysis to remove RNA and column purification (Zymo Research). The final probes were resuspended in RNAase-free water and stored at -20 °C.

Oligo conjugation to secondary antibodies. Oligonucleotides containing the desired readout probes were conjugated to secondary antibodies via a combination of NHS-ester and copper-free click chemistries. First, secondary antibodies were labeled with a copper-free click crosslinking agent using NHS-ester chemistry. Specifically, azide preservative was removed from the unconjugated Donkey Anti-Rabbit secondary antibodies (Thermoscientific) using a spin-column based dialysis membrane (Amicon, 100kDa molecular weight cut off) according to the manufacturer's instructions. The NHS-ester, PEG5, DBCO cross linker (Kerafast) was diluted to a concentration of 10 micromolar in anhydrous DMSO (Thermoscientific). 2 microliters of this cross linker was then combined with 100 microliters of 2 mg/mL of the antibody in 1x phosphate-buffered saline (PBS). This reaction was incubated at room temperature for 1 hour and then terminated via a second round of purification using the Amicon columns as described above. The average number of DBCO crosslinkers per antibody was determined via the relative absorption of the sample at 280 nm (antibody) and 309 nm (DBCO). On average the crosslinker amounts described above produced ~7 DBCO crosslinkers per antibody.

Oligonucleotide probes containing the desired sequence as well as a 5'-acrydite, to allow cross linking to the polymer gel, and a 3'-azide, to allow crosslinking to the DBCO-labeled antibodies, were ordered from IDT and suspended to 100 micromolar in 1x PBS. 20 microliters of the appropriate oligonucleotide was then added to 100 microliters of the DBCO-labeled antibodies at a final concentration of ~2 mg/mL. This reaction was incubated at 4 °C for at least 12 hours. Labeled antibodies were not further purified as residual oligonucleotides, not conjugated to antibodies, are expected to be readily washed away from samples.

Cell culture and fixation. U-2 OS cells (ATCC) were cultured with Eagle's Minimum Essential Medium (ATCC) containing 10% (vol/vol) FBS (Thermo Fisher Scientific). Cells were plated on 40-mm-diameter, no. 1.5 coverslips (Bioptechs) at 350,000 cells per coverslip and were incubated in Petri dishes at 37 °C with 5% $CO_2$ for 48 h. Cells were fixed, permeabilized, and stained with encoding probes. Briefly, cells were fixed for 15 min in 4% (vol/vol) paraformaldehyde (Electron Microscopy Sciences) in 1x PBS at room temperature, washed three times with 1x PBS, permeabilized for 10 min with 0.5% (vol/vol) Triton X-100 (Sigma) in 1x PBS at room temperature, and washed once with 1x PBS.

Encoding probe staining for MERFISH measurement. Permeabilized cells were incubated for 5 min in encoding wash buffer comprising 2x saline-sodium citrate (SSC) (Ambion) and 30% (vol/vol) formamide (Ambion). Then 30 microliters of ~300 micromolar encoding probes and 3.3 micromolar of anchor probe (a 20-nt sequence of alternating dT and thymidine-locked nucleic acid (dT+) with a 20-nt reverse complement of a readout sequence and a 5'-acrydite modification (Integrated DNA Technologies)) in encoding hybridization buffer was added to the surface of Parafilm (Bemis) and was covered with a cell-containing coverslip. Samples then were incubated in a humid chamber inside a hybridization oven at 37 °C for 40 h. Encoding hybridization buffer was composed of encoding wash buffer supplemented with 0.1% (wt/vol) yeast tRNA (Life Technologies), 1% (vol/vol) murine RNase inhibitor (New England Biolabs), and 10% (wt/vol) dextran sulfate (Sigma). Cells then were washed with encoding wash buffer and were incubated at 47 °C for 30 min; this washing step was repeated once.

Immunostaining. After being stained with encoding and anchor probes, samples were post-fixed with 4% (vol/vol) paraformaldehyde (Electron Microscopy Sciences) at room temperature for 10 min. Samples were then blocked at room temperature for 30 min in blocking buffer with 4% (wt/vol) UltraPure BSA (Thermo Fisher Scientific) in 2x SSC (Ambion) supplemented with 3% (vol/vol) RNasin Ribonuclease inhibitors (Promega), 6% (vol/vol) murine RNAase inhibitors (New England Biolabs) and 1 mg/ml yeast tRNA (Life Technologies). Samples were incubated with primary antibodies (anti-pan Cadherin, Abcam) in blocking buffer at a concentration of 2 micrograms/ml for 1 h at room temperature, and washed three times with 2x SSC for 10 min each. Samples were then incubated with oligo-labeled secondary antibodies in blocking buffer at a concentration of 3.75 micrograms/ml for 1 h at room temperature, then washed with 2x SSC three times for 10 min each. Samples were fixed again with 4% (vol/vol) paraformaldehyde (Electron Microscopy Sciences) for 10 min.

Embedding, digestion and clearing for unexpanded samples. Stained samples on coverslips were first incubated for 5 min with a de-gassed polyacrylamide solution with 4% (vol/vol) of 19:1 acrylamide/bis-acrylamide (BioRad), 60 mM Tris·HCl pH 8 (ThermoFisher), 0.3 M NaCl (ThermoFisher), 0.2% (vol/vol) Tetramethylethylenediamine (TEMED) and a 1:25,000 dilution of 0.1-micrometer-diameter carboxylate-modified orange fluorescent beads (Life Technologies). The beads served as fiducial markers for the alignment of images taken across multiple rounds of smFISH imaging.

To cast a thin polyacrylamide film, for each sample, 50 microliters of the final solution was added to the surface of a glass plate (TED Pella) that had been pretreated for 5 min with 1 mL GelSlick (Lonza) so as not to stick to the polyacrylamide gel. Samples on coverslips, treated as described above, were aspirated and gently inverted onto this 50-microliter droplet to form a thin layer of solution between the coverslip and the glass plate. The solution was then allowed to polymerize for 2 h at room temperature in a home-built chamber filled with nitrogen. The coverslip and the glass plate were then gently separated, and the PA film was washed once with a digestion buffer with 2% (wt/vol) SDS (Thermo Fisher Scientific), 0.5% (vol/vol) Triton X-100 in 2x SSC (Ambion). The digestion buffer used 2% (wt/vol) SDS to facilitate lipid removal. After the wash, the gel was covered with digestion buffer supplemented with 1% (vol/vol) Proteinase K (New England Biolabs). The sample was digested in this buffer for >12 h in a humidified, 37 °C incubator and then washed three times with 2x SSC. MERFISH measurements were either performed immediately or the sample was stored in 2x SSC supplemented with 0.1% (vol/vol) murine RNase inhibitor (New England Biolabs) at 4 °C for no longer than 48 h.

Silanization of coverslips for unexpanded samples. 40-mm-diameter No. 1.5 coverslips (Bioptechs) were washed for 30 min via immersion in a 1:1 mixture of 37% (vol/vol) HCl and methanol at room temperature. The coverslips were then rinsed three times in deionized water and once in 70% (vol/vol) ethanol. The coverslips were blown dry with nitrogen gas and then immersed in 0.1% (vol/vol) triethylamine (Millipore) and 0.2% (vol/vol) allyltrichlorosilane (Sigma) in chloroform for 30 min at room temperature. The coverslips were washed once each with chloroform and ethanol and then blown dry with nitrogen gas. Silanized coverslips were then stored at room temperature in a desiccated chamber overnight before use, to dehydrate the silane layer.

Embedding, digestion and clearing for expanded samples. The monomer solution containing 2 M NaCl (Thermo Fisher Scientific), 7.7% (wt/wt) sodium acrylate (Sigma), 4% (vol/vol) of 19:1 acrylamide/bis-acrylamide (BioRad) and 60 mM Tris HCl pH 8 (Thermo Fisher Scientific) was prepared, frozen in aliquots at -20 °C, and thawed before use. The monomer solution was degassed and cooled to 4 °C before use. TEMED with a final concentration of 0.2% (vol/vol) and a 1:5,000 dilution of 0.1-micrometer-diameter carboxylate-modified orange fluorescent beads (Life Technologies) was added to the solution. The beads served as fiducial markers for the alignment of images taken across multiple rounds of smFISH imaging. Stained samples were incubated in the solution for 5 min at room temperature. The solution was then kept on ice and further supplemented with ammonium persulfate (Sigma) at a final concentration of 0.2% (wt/vol).

To cast a thin polymer film, for each sample, 50 microliters of the final solution was added to the surface of a glass plate (TED Pella) that had been pretreated for 5 min with 1 mL GelSlick (Lonza) so as not to stick to the gel. Samples on coverslips, treated as described above, were aspirated and gently inverted onto this 50-microliter droplet to form a thin layer of solution between the coverslip and the glass plate. The solution was then allowed to polymerize for 2 h at room temperature in a home-built chamber filled with nitrogen. The coverslip and the glass plate were then gently separated, and the gel was washed once with a digestion buffer with 2% (wt/vol) SDS (Thermo Fisher Scientific), 0.5% (vol/vol) Triton X-100 in 2x SSC (Ambion) as described above. After the wash, the thin film of the gel were carefully trimmed to desired sizes using a razor blade, and covered with digestion buffer supplemented with 1% (vol/vol) Proteinase K (New England Biolabs). The sample was digested in this buffer for >12 h in a humidified, 37 °C incubator and then washed with 2x SSC (Ambion) three times. The gel would expand -1.5 fold during digestion.

Expansion and re-embedding. After digestion, samples were expanded in 0.5x SSC buffer supplemented with 0.2% (vol/vol) Proteinase K (New England Biolabs) at room temperature. Proteinase K was added to maintain samples in an RNAase free environment. The buffer was changed every 30 min until samples no longer expanded (typically about 2 h). Expanded gels were re-embedded in polyacrylamide gel to stabilize the gel for sequential rounds of readout probe hybridization and imaging. Briefly, samples were incubated in re-embedding solution composed of 4% (vol/vol) of 19:1 acrylamide/bis-acrylamide (BioRad)) with 30 mM NaCl (Thermo Fisher Scientific), 6 mM Tris HCl pH 8 (Thermo Fisher Scientific) and 0.2% (vol/vol) of TEMED (Sigma) for 20 min at room temperature on a rocker. The re-embedding solution was then kept on ice and further supplemented with ammonium persulfate (Sigma) at a final concentration of 0.2% (wt/vol). Gels were placed on a bind-silane-treated coverslip (see below), rinsed with the solution and dried quickly with KimWipes (Kimtech). Coverslips with gels were put in a home-built nitrogen chamber, covered a glass plate (TED Pella) and allowed to polymerize at room temperature for 1 h.

Bind-silane treatment of coverslips. 40-mm-diameter, no. 1.5 coverslips (Bioptechs) were sonicated in 1 M potassium hydroxide for 30 min, wash three times with deionized water and sonicated again in 70% (vol/vol) ethanol for 30 min. The coverslips were then immersed in 5% (vol/vol) glacial acetic acid and 0.38% (vol/vol) bind-silane (GE Healthcare) in 99% (vol/vol) ethanol for 1 h at room temperature. After being quickly washed with 70% (vol/vol) ethanol three times, the coverslips were put into a 60 °C oven until dried completely. Coverslips can be stored in seal containers with desiccants for up to a month.

Sequential rounds of readout probe staining and imaging. To aid choosing the right focal plane for imaging, embedded samples were hybridized in dish with the first pair of two-color readout probes in hybridization buffer composed of 2x SSC (Ambion), 5% (vol/vol) ethylene carbonate (Sigma), 0.1% (vol/vol) murine RNase inhibitor in nuclease-free water, and 3 nM of the appropriate readout probes, for 30 min (expanded samples) or 10 min (unexpanded samples) at room temperature and washed for 20 min (expanded samples) or 7 min (unexpanded samples) in wash buffer composed of 2x SSC (Ambion) and 10% (vol/vol) ethylene carbonate (Sigma) in nuclease-free water. Samples were then washed with 2x SSC once, stained with DAPI at 10 micrograms/microliter in 2x SSC (Ambion) for 10 min, and washed 3 times in 2x SSC (Ambion) for 5 min each.

Samples were mounted into a flow chamber and the buffer exchange through this chamber for following imaging and hybridization was controlled via a home-built fluidics system composed of three computer-controlled eight-way valves (Hamilton) and a computer-controlled peristaltic pump (Gilson), with elongated flow and incubation time for expanded samples (besides the hybridization and wash time as described in the previous section. Tris(2-carboxyethyl)phosphine hydrochloride (TCEP) incubation time was increased to 30 min and buffer exchange time to 7 min) to allow diffusion to reach equilibrium inside of the gel.

In order to accurately compare the counts per cell between different samples, more than 90% of target RNAs in a cell should be caught. To quantify RNA distribution across different z planes, optical sectioning was performed at discrete imaging planes across the cell at a step size of 0.5 micrometers and the distribution of smFISH signals was quantified as a function of z position. It was found that >90% of RNAs located in the first 5 micrometer-depth volume in unexpanded samples and in the first 12 micrometer-depth volume in expanded samples from the surface of coverslips. Thus, a 5 micrometer-depth volume was scanned for unexpanded samples and a 12 micrometer-depth volume was scanned for expanded samples with a step size of 1 micrometer. The scanning in z direction was controlled by a Nano-F200 nanopositioner (Mad City Labs).

Sequential MERFISH imaging and signal removal was carried out on a high-throughput imaging platform. Briefly, after hybridization, samples were imaged with a FOV area of 223 x 223 micrometers utilizing a 2,048 x 2,048 pixel, scientific complementary metal-oxide semiconductor (sCMOS) camera in combination with a high numerical aperture (NA = 1.3) and a high-magnification (60x) silicone oil objective. After imaging -100-400 FOVs, the fluorescence of the readout probes was extinguished by incubating the sample in a reductive cleavage buffer composed of 2x SSC and 50 mM TCEP (Sigma). The hybridization, imaging and chemical cleavage process was repeated eight times with 405-nm DAPI channel imaged in conjunction with the first round of readout imaging.

Image Registration and Decoding. Registration of images of the same FOV across imaging rounds as well as decoding of the RNA barcodes was conducted as follows. Briefly, z-stacks at each location from different imaging rounds were corrected for lateral offsets based on the location of fiducial beads. Corrected z-stacks were high-pass filtered to remove background, deconvolved to tighten RNA spots, and then low-pass filtered to connect RNA centroids that differed slightly in location between images. To correct for differences in the brightness between color channels, images were first normalized by equalizing their intensity histograms and refined further via an iterative process to remove substantial variation in the fluorescence intensity between different bits. The set of 16 normalized intensity values (corresponding to 16 readout probe imaging) observed for each pixel in each FOV at each focal plane represented a vector in a 16-dimensional space. The pixel vector was normalized and compared to each of the 140 barcodes in the 16-bit MHD4 code. A pixel was assigned to a barcode if the Euclidean distance between the vector and a barcode was smaller than a given threshold defined by the distance of a single-bit error. Adjacent pixels were combined into a single putative RNA using a 3-D connectivity array with maximal neighborhood connectivity. Cell boundaries were calculated using the watershed algorithm based on the inverted barcode density with DAPI- stained regions as initial seeds.

Computations were split between the Odyssey cluster supported by the FAS Division of Science, Research Computing Group at Harvard University and a desktop server that contained two 10-core Intel Xeon E5-2680 2.8GHz CPUs and 256 GB of RAM.

Single-molecule FISH. Each smFISH probe contains a 30-nt target region and a 20-nt custom-designed readout sequence. 48 probes were designed for each gene. After permeabilization, cells were incubated for 5 min in encoding wash buffer comprising 2x saline-sodium citrate (SSC) (Ambion) and 30% (vol/vol) formamide (Ambion). Then 30 microliters of 2 micromolar smFISH probes and 3.3 micromolar of anchor probe in encoding hybridization buffer was added to the surface of Parafilm (Bemis) and was covered with a cell-containing coverslip. Samples then were incubated in a humid chamber inside a hybridization oven at 37 °C for 24 h. Encoding hybridization buffer was composed of encoding wash buffer supplemented with 0.1% (wt/vol) yeast tRNA (Life Technologies), 1% (vol/vol) murine RNase inhibitor (New England Biolabs), and 10% (wt/vol) dextran sulfate (Sigma). Cells then were washed with encoding wash buffer and were incubated at 47 °C for 30 min; this washing step was repeated once. Cells were then embedded and cleared using the matrix-imprinting-based clearing method described as described above for embedding, digestion and clearing for unexpanded samples. Embedded samples were hybridized in dish with a Cy5 20-nt readout probes in hybridization buffer, washed and stained with DAPI as described in Sequential rounds of readout probe staining and imaging Section. Images were collected scanning of a 5 micrometer-depth volume at a step size of 1 micrometer and 100 FOVs were collected for each smFISH probe set.

While several embodiments of the present invention have been described and illustrated herein, those of ordinary skill in the art will readily envision a variety of other means and/or structures for performing the functions and/or obtaining the results and/or one or more of the advantages described herein, and each of such variations and/or modifications is deemed to be as defined by the claims. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used. Those skilled in the art will recognize, or be able to ascertain using no more than routine experimentation, many equivalents to the specific embodiments of the invention described herein. It is, therefore, to be understood that the foregoing embodiments are presented by way of example only and that, within the scope of the appended claims, the invention may be practiced otherwise than as specifically described. The present disclosure is directed to each individual feature, system, article, material, kit, and/or method described herein. In addition, any combination of two or more such features, systems, articles, materials, kits, and/or methods, if such features, systems, articles, materials, kits, and/or disclosure methods are not mutually inconsistent, is included within the scope of the present invention.

All definitions, as defined and used herein, should be understood to control over dictionary definitions, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

When the word "about" is used herein in reference to a number, it should be understood that still another embodiment of the invention includes that number not modified by the presence of the word "about."

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03.

**Claims**

1. A method, comprising:

   embedding cells within an expandable material, the expandable material comprising acrylates and/or acrylamides;

immobilizing target nucleic acids from the cells to the expandable material, comprising exposing the target nucleic acids to a plurality of anchor probes that immobilize the target nucleic acids at a single region of each target nucleic acid to the expandable material, wherein each of the plurality of anchor probes comprise a nucleic acid sequence complementary to at least a portion of the target nucleic acids from the cells and a portion that reacts with the expandable material;

expanding the expandable material, comprising exposing the expandable material to a solution comprising water and/or to a solution hypotonic to the expandable material;

exposing the expandable material to a plurality of nucleic acid probes; and

determining binding of the nucleic acid probes to the immobilized target nucleic acids.

2. The method of claim 1, comprising immobilizing the nucleic acids at a region located at:

(a) a 5' end of the nucleic acids; or
(b) a 3' end of the nucleic acids; or
(c) a central portion of the nucleic acids.

3. The method of any one of claims 1-2,

(a) wherein a unique anchor probe is used for each target nucleic acid, or wherein one or more unique anchor probes are used for each target nucleic acid; and/or
(b) wherein at least some of the plurality of anchor probes comprise a poly-dT.

4. The method of any one of claims 1-3, wherein at least some of the plurality of anchor probes comprise an acrydite-modified oligonucleotide.

5. The method of any one of claims 1-4, wherein embedding cells within an expandable material comprises surrounding the cells with a precursor of the expandable material, and causing the precursor of the expandable material to form the expandable material surrounding the cells, preferably wherein:

(a) the precursor of the expandable material comprises a monomer; and/or
(b) causing the precursor to form the expandable material comprises polymerizing the precursor; and/or
(c) causing the precursor to form the expandable material comprises exposing the precursor to a cross-linking agent.

6. The method of any one of claims 1-5, wherein the precursor comprises acrylamide and/or an acrylate.

7. The method of any one of claims 1-6, wherein the expandable material comprises a gel and/or polyacrylamide and/or methacrylate.

8. The method of any one of claims 1-7, comprising expanding the expandable material isotopically.

9. The method of any one of claims 1-8, further comprising fixing the cells, preferably wherein fixing the cells comprises exposing the cells to paraformaldehyde.

10. The method of any one of claims 1-9, further comprising, for each at least some of the plurality of nucleic acid probes, determining binding of the nucleic acid probes within the sample.

11. The method of any one of claims 1-10, further comprising creating codewords based on the binding of the plurality of nucleic acid probes; and for at least some of the codewords, matching the codeword to a valid codeword wherein, if no match is found, applying error correction to the codeword to form a valid codeword.

12. The method of any one of claims 1-11, comprising exposing the cells to:

(a) at least 5 different nucleic acid probes; and/or
(b) the plurality of nucleic acid probes simultaneously; and/or
(c) the plurality of nucleic acid probes sequentially.

13. The method of any one of claims 1-12, wherein the plurality of nucleic acid probes comprises a combinatorial combination of nucleic acid probes with different sequences.

14. The method of any one of claims 1-13, wherein at least some of the plurality of nucleic acid probes comprises a first portion comprising a target sequence and a second portion comprising one or more read sequences, preferably:

(a) wherein the plurality of nucleic acid probes comprises distinguishable nucleic acid probes formed from combinatorial combination of one or more read sequences taken from the one or more read sequences; and/or
(b) comprising exposing the cells to a first secondary probe comprising a first signaling entity, the first secondary probe able to bind to some of the read sequences of the nucleic acid probes, and determining binding of the nucleic acid probes by determining the first signaling entity, preferably further comprising exposing the cells to a second secondary probe comprising a second signaling entity, the second secondary probe able to bind to some of read sequences of the nucleic acid probes, and determining binding of the nucleic acid probes by determining the second signaling entity.

15. The method of any one of claims 1-14, comprising determining binding of the nucleic acid probes within the sample:

(a) at a resolution better than 300 nm after expansion of the expandable material, preferably better than 100 nm; and/or
(b) at an effective resolution better than 30 nm prior to expansion of the expandable material, preferably better than 10 nm; and/or
(c) by imaging at least a portion of the expandable material; and/or
(d) using an optical imaging technique; and/or
(e) using a fluorescence imaging technique; and/or
(f) using a super-resolution fluorescence imaging technique.

**Patentansprüche**

1. Verfahren, umfassend:

Einbetten von Zellen in ein expandierbares Material, das expandierbare Material umfassend Acrylate und/oder Acrylamide;
Immobilisieren von Zielnukleinsäuren aus den Zellen zu dem expandierbaren Material, umfassend Aussetzen der Zielnukleinsäuren gegenüber einer Vielzahl von Ankerproben, die die Zielnukleinsäuren an einer einzelnen Region jeder Zielnukleinsäure an dem expandierbaren Material immobilisieren, wobei jede der Vielzahl von Ankerproben eine Nukleinsäuresequenz umfasst, die komplementär zu mindestens einem Teil der Zielnukleinsäuren aus den Zellen und einem Teil, der mit dem expandierbaren Material reagiert, ist;
Expandieren des expandierbaren Materials, umfassend Aussetzen des expandierbaren Materials gegenüber einer Wasser umfassenden Lösung und/oder einer für das expandierbare Material hypotonischen Lösung;
Exponieren des expandierbaren Materials gegenüber einer Vielzahl von Nukleinsäureproben; und
Bestimmen der Bindung der Nukleinsäureproben an die immobilisierten Zielnukleinsäuren.

2. Verfahren gemäß Anspruch 1, umfassend Immobilisieren der Nukleinsäuren in einem Bereich, der sich an:

(a) einem 5'-Ende der Nukleinsäuren; oder
(b) einem 3'-Ende der Nukleinsäuren; oder
(c) einem zentralen Abschnitt der Nukleinsäuren, befindet.

3. Verfahren gemäß irgendeinem der Ansprüche 1-2,

(a) wobei eine einzigartige Ankerprobe für jede Zielnukleinsäure verwendet wird oder wobei eine oder mehrere einzigartige Ankerproben für jede Zielnukleinsäure verwendet werden; und/oder
(b) wobei mindestens einige der Vielzahl von Ankerproben ein poly-dT umfassen.

4. Verfahren gemäß irgendeinem der Ansprüche 1-3, wobei mindestens einige der mehreren Ankerproben ein Acryditmodifiziertes Oligonukleotid umfassen.

5. Verfahren gemäß irgendeinem der Ansprüche 1-4, wobei Einbetten von Zellen in ein expandierbares Material das Umgeben der Zellen mit einem Vorläufer des expandierbaren Materials und das Bewirken, dass der Vorläufer des expandierbaren Materials das die Zellen umgebende expandierbare Material bildet, umfasst, wobei vorzugsweise

(a) der Vorläufer des expandierbaren Materials ein Monomer umfasst; und/oder
(b) Bewirken des Vorläufers, das expandierbare Material zu bilden, das Polymerisieren des Vorläufers umfasst; und/oder
(c) Bewirken des Vorläufers, das expandierbare Material zu bilden, Exponieren des Vorläufers gegenüber einem Vernetzungsmittel umfasst.

6. Verfahren gemäß irgendeinem der Ansprüche 1-5, wobei der Vorläufer Acrylamid und/oder ein Acrylat umfasst.

7. Verfahren gemäß irgendeinem der Ansprüche 1-6, wobei das expandierbare Material ein Gel und/oder Polyacrylamid und/oder Methacrylat umfasst.

8. Verfahren gemäß irgendeinem der Ansprüche 1-7, umfassend dem isotopisch expandieren des expandierbaren Materials.

9. Verfahren gemäß irgendeinem der Ansprüche 1-8, ferner umfassend Fixieren der Zellen, vorzugsweise wobei Fixieren der Zellen das Aussetzen der Zellen gegenüber Paraformaldehyd umfasst.

10. Verfahren gemäß irgendeinem der Ansprüche 1-9, ferner umfassend für zumindest einige der mehreren Nukleinsäureproben die Bestimmung der Bindung der Nukleinsäureproben innerhalb der Probe.

11. Verfahren gemäß irgendeinem der Ansprüche 1-10, ferner umfassend Erzeugen von Codewörtern auf der Grundlage der Bindung der Vielzahl von Nukleinsäureproben; und für mindestens einige der Codewörter, Zuordnen des Codewortes an ein gültiges Codewort, wobei, wenn keine Übereinstimmung gefunden wird, eine Fehlerkorrektur auf das Codewort angewendet wird, um ein gültiges Codewort zu bilden.

12. Verfahren gemäß irgendeinem der Ansprüche 1-11, umfassend Aussetzen der Zellen gegenüber:

(a) mindestens 5 verschiedenen Nukleinsäureproben; und/oder
(b) der Vielzahl von Nukleinsäureproben gleichzeitig; und/oder
(c) der Vielzahl von Nukleinsäureproben nacheinander.

13. Verfahren gemäß irgendeinem der Ansprüche 1-12, wobei die Vielzahl von Nukleinsäureproben eine kombinatorische Kombination von Nukleinsäureproben mit unterschiedlichen Sequenzen umfasst.

14. Verfahren gemäß irgendeinem der Ansprüche 1-13, wobei mindestens einige der Vielzahl von Nukleinsäureproben einen ersten Teil mit einer Zielsequenz und einen zweiten Teil mit einer oder mehreren Lesesequenzen umfasst, vorzugsweise:

(a) wobei die Vielzahl von Nukleinsäureproben unterscheidbare Nukleinsäureproben umfasst, die aus einer kombinatorischen Kombination von einer oder mehreren Lesesequenzen gebildet sind, die aus der einen oder den mehreren Lesesequenzen entnommen wurden; und/oder
(b) umfassend Aussetzen der Zellen gegenüber einer ersten sekundären probe, die eine erste signalgebende Entität umfasst, wobei die erste sekundäre Probe in der Lage ist, an einige der Lesesequenzen der Nukleinsäureproben zu binden, und das Bestimmen der Bindung der Nukleinsäureproben durch Bestimmen der ersten signalgebenden Entität, vorzugsweise weiterhin umfassend das Aussetzen der Zellen einer zweiten sekundären Probe, die eine zweite signalgebende Entität umfasst, wobei die zweite sekundäre Probe in der Lage ist, an einige der Lesesequenzen der Nukleinsäureproben zu binden, und das Bestimmen der Bindung der Nukleinsäureproben durch Bestimmen der zweiten signalgebenden Entität.

15. Verfahren gemäß irgendeinem der Ansprüche 1-14, umfassend Bestimmen der Bindung der Nukleinsäureproben in der Probe:

(a) mit einer Auflösung von besser als 300 nm nach der Expansion des expandierbaren Materials, vorzugsweise besser als 100 nm; und/oder
(b) mit einer effektiven Auflösung von besser als 30 nm vor der Expansion des expandierbaren Materials, vorzugsweise besser als 10 nm; und/oder
(c) durch Abbildung mindestens eines Teils des expandierbaren Materials; und/oder
(d) unter Verwendung einer optischen Abbildungstechnik; und/oder
(e) unter Verwendung einer Fluoreszenzabbildungstechnik; und/oder
(f) unter Verwendung eines Fluoreszenzabbildungsverfahrens mit Superauflösung.

**Revendications**

1. Procédé, comprenant :

l'intégration de cellules dans un matériau expansible, le matériau expansible comprenant des acrylates et/ou acrylamides ;
l'immobilisation d'acides nucléiques cibles des cellules sur le matériau expansible, comprenant l'exposition des acides nucléiques cibles à une pluralité de sondes d'ancrage qui immobilisent les acides nucléiques cibles au niveau d'une région unique de chaque acide nucléique cible sur le matériau expansible, dans lequel chacune de la pluralité de sondes d'ancrage comprend une séquence d'acide nucléique complémentaire d'au moins une partie des acides nucléiques cibles provenant des cellules et une partie qui réagit avec le matériau expansible ;
l'expansion du matériau expansible, comprenant l'exposition du matériau expansible à une solution comprenant de l'eau et/ou à une solution hypotonique pour le matériau expansible ;
l'exposition du matériau expansible à une pluralité de sondes d'acide nucléique ; et la détermination de la liaison des sondes d'acide nucléique aux acides nucléiques cibles immobilisés.

2. Procédé selon la revendication 1, comprenant l'immobilisation des acides nucléiques au niveau d'une région située à :

(a) une extrémité 5' des acides nucléiques ; ou
(b) une extrémité 3' des acides nucléiques ; ou
(c) une partie centrale des acides nucléiques.

3. Procédé selon l'une quelconque des revendications 1 et 2,

> (a) dans lequel une sonde d'ancrage unique est utilisée pour chaque acide nucléique cible, ou dans lequel une ou plusieurs sondes d'ancrage uniques sont utilisées pour chaque acide nucléique cible ; et/ou
> (b) dans lequel au moins certaines de la pluralité de sondes d'ancrage comprennent un poly-dT.

4. Procédé selon l'une quelconque des revendications 1 à 3, dans lequel au moins certaines de la pluralité de sondes d'ancrage comprennent un oligonucléotide modifié par de l'acrydite.

5. Procédé selon l'une quelconque des revendications 1 à 4, dans lequel l'intégration de cellules dans un matériau expansible comprend le fait d'entourer les cellules avec un précurseur du matériau expansible, et d'amener le précurseur du matériau expansible à former le matériau expansible entourant les cellules, de préférence dans lequel :

> (a) le précurseur du matériau expansible comprend un monomère ; et/ou
> (b) le fait d'amener le précurseur à former le matériau expansible comprend la polymérisation du précurseur ; et/ou
> (c) le fait d'amener le précurseur à former le matériau expansible comprend l'exposition du précurseur à un agent de réticulation.

6. Procédé selon l'une quelconque des revendications 1 à 5, dans lequel le précurseur comprend de l'acrylamide et/ou un acrylate.

7. Procédé selon l'une quelconque des revendications 1 à 6, dans lequel le matériau expansible comprend un gel et/ou du polyacrylamide et/ou du méthacrylate.

8. Procédé selon l'une quelconque des revendications 1 à 7, comprenant l'expansion de manière isotopique du matériau expansible.

9. Procédé selon l'une quelconque des revendications 1 à 8, comprenant en outre la fixation des cellules, de préférence dans lequel la fixation des cellules comprend l'exposition des cellules à du paraformaldéhyde.

10. Procédé selon l'une quelconque des revendications 1 à 9, comprenant en outre, pour chacune d'au moins certaines de la pluralité de sondes d'acide nucléique, la détermination de la liaison des sondes d'acide nucléique dans l'échantillon.

11. Procédé selon l'une quelconque des revendications 1 à 10, comprenant en outre la création de mots de code basés sur la liaison de la pluralité de sondes d'acide nucléique ; et pour au moins certains des mots de code, le fait de faire correspondre le mot de code à un mot de code valide et, si aucune correspondance n'est trouvée, l'application d'une correction d'erreur au mot de code pour former un mot de code valide.

12. Procédé selon l'une quelconque des revendications 1 à 11, comprenant l'exposition des cellules à :

> (a) au moins 5 sondes d'acide nucléique différentes ; et/ou
> (b) la pluralité de sondes d'acide nucléique simultanément ; et/ou
> (c) la pluralité de sondes d'acide nucléique séquentiellement.

13. Procédé selon l'une quelconque des revendications 1 à 12, dans lequel la pluralité de sondes d'acide nucléique comprend une combinaison combinatoire de sondes d'acide nucléique avec des séquences différentes.

14. Procédé selon l'une quelconque des revendications 1 à 13, dans lequel au moins certaines de la pluralité de sondes d'acide nucléique comprennent une première partie comprenant une séquence cible et une seconde partie comprenant une ou plusieurs séquences lues, de préférence :

> (a) dans lequel la pluralité de sondes d'acide nucléique comprend des sondes d'acide nucléique distinctes formées à partir d'une combinaison combinatoire d'une ou plusieurs séquences lues extraites de la ou des séquences lues ; et/ou
> (b) comprenant l'exposition des cellules à une première sonde secondaire comprenant une première entité de signalisation, la première sonde secondaire étant capable de se lier à certaines des séquences lues des sondes d'acide nucléique, et la détermination de la liaison des sondes d'acide nucléique en déterminant la première entité de signalisation, de préférence comprenant en outre l'exposition des cellules à une seconde sonde secondaire comprenant une seconde entité de signalisation, la seconde sonde secondaire étant capable de se lier à certaines des séquences lues des sondes d'acide nucléique, et la détermination de la liaison des sondes d'acide nucléique en déterminant la seconde entité de signalisation.

15. Procédé selon l'une quelconque des revendications 1 à 14, comprenant la détermination de la liaison des sondes d'acide nucléique au sein de l'échantillon :

(a) à une résolution meilleure que 300 nm après l'expansion du matériau expansible, de préférence meilleure que 100 nm ; et/ou

(b) à une résolution effective meilleure que 30 nm avant l'expansion du matériau expansible, de préférence meilleure que 10 nm ; et/ou

(c) par l'imagerie d'au moins une partie du matériau expansible ; et/ou

(d) en utilisant une technique d'imagerie optique ; et/ou

(e) en utilisant une technique d'imagerie par fluorescence ; et/ou

(f) en utilisant une technique d'imagerie par fluorescence à super-résolution.

FIG. 1A

FIG. 1B

FIG. 1C

FIG. 1D

FIG. 1E

FIG. 2A

FIG. 2B

FIG. 2C

FIG. 2D

FIG. 2E

FIG. 2F

FIG. 2G

FIG. 2H

FIG. 3A

FIG. 3B

FIG. 3C

FIG. 3D

FIG. 3E

mean ratio: 0.99 ± 0.03
median ratio: 0.94
$\rho_{10} = 0.95$

count / cell
(expanded + IF)

count / cell
(expanded)

FIG. 3F

30 μm

FIG. 3G

$\times 10^6$

intensity / um (a.u.)

control　　Expansion
MERFISH

FIG. 3H

FIG. 4A

FIG. 4B

FIG. 4C

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62419033, Zhuang, et al.
- US 62569127, Zhuang
- US 2016305856 A1
- US 2015144490 A1
- US 2015087001 A1
- US 2016304952 A1
- US 329683, Zhuang
- US 20170220733
- US 7838302 B, Zhuang
- US 32968317, Zhuang
- US 32965117, Zhuang
- US 20170212986
- WO 2016018960 A
- WO 2016018963 A
- US 8564792 B, Zhuang
- WO 2013090360 A, Zhuang
- US 61979436 B
- US 2007017618 W, Zhuang
- WO 2008091296 A
- US 7776613 B, Zhuang
- US 2015042559 W, Zhuang
- US 2015042556 W, Zhuang
- US 25230716, Zhuang
- US 20160370295
- US 62511920, Zhuang
- US 20170220733 A
- US 20170212986 A

**Non-patent literature cited in the description**

- **FEI CHEN et al.** *Nature Methods,* 2016, vol. 13 (8), 679-684
- **NIKOLAY TSANOV et al.** *Nucleic Acids Research,* 2016, vol. 44 (22), e165-e165
- **FEI CHEN et al.** *Science,* 2015, vol. 347 (6221), 543-548
- **FEI CHEN et al.** *Science,* 2015, vol. 347 (6221), 1-18